# FASCICULE DE BREVET EUROPEEN

(11) **EP 0 834 508 B1**
(45) Date de publication et mention de la délivrance du brevet: **16.08.2001**
(21) Numéro de dépôt: 97402321.0
(22) Date de dépôt: 03.10.1997
(51) Int. Cl.: C07D 307/79, C07D 213/61, C07D 209/08, C07D 405/06, C07D 405/04, C07D 409/06, C07C 223/06, C07C 251/48, C07C 255/53, C07C 291/02, C07C 47/57, C07C 205/20, A61K 31/34, A61K 31/44, A61K 31/15

(54) **Nouveaux dérivés substitués de biphényle ou de phénylpyridine, leur procédé de préparation et les compositions pharmaceutiques qui les contiennent**
Substituierte Biphenyl- oder Phenylpyridinderivate, Verfahren zu deren Herstellung und sie enthaltende pharmazeutische Zusammensetzungen
Substituted biphenyl or phenylpyridine derivatives, process for their preparation and pharmaceutical compositions containing them

(30) Priorité: 04.10.1996 FR 9612111
(43) Date de publication de la demande: 08.04.1998
(73) Titulaire: ADIR ET COMPAGNIE, 92415 Courbevoie Cédex (FR)
(72) Inventeur: Dhainaut, Alain, 78400 Chatou (FR); Poissonnet, Guillaume, 91120 Villebon sur Yvette (FR); Canet, Emmanuel, 75005 Paris (FR); Lonchampt, Michel, 94150 Chevilly La Rue (FR)

(56) Documents cités:
- WO-A-96/03396
- FR-A- 2 142 470

## Description

La présente invention concerne de nouveaux dérivés substitués de biphényle ou de phénylpyridine, leur procédé de préparation et les compositions pharmaceutiques qui les contiennent. Ces composés sont des inhibiteurs de phosphodiestérases du groupe IV et de ce fait présentent des applications thérapeutiques particulièrement intéressantes.

Les fonctions de la plupart des tissus organiques sont modulées par des substances endogènes (hormones, neurotransmetteurs, autacoïdes) ou exogènes. Pour certaines de ces substances, l'effet biologique est relayé au niveau intracellulaire par des effecteurs enzymatiques tels que l'adénylate cyclase ou la guanylate cyclase. La stimulation de ces enzymes responsables de la synthèse de nucléotides cycliques tels que l'adenosine-3',5'-monophosphate cyclique (AMPc) et la guanosine-3',5'-monophosphate cyclique (GMPc) entraîne une élévation du taux intracellulaire de ces seconds messagers impliqués dans la régulation de nombreuses fonctions biologiques (E. W. SUTHERLAND et T. W. RALL, Pharmacol. Rev., Vol. 12, p. 265, 1960). La dégradation des nucléotides cycliques est assurée par une famille d'enzymes, appelées phosphodiestérases (PDE), classées actuellement en 7 groupes. La reconnaissance d'isoformes différentes à l'intérieur de chacun de ces groupes, et de la distribution tissu- ou cellule-spécifique de certaines isoformes , a stimulé la recherche d'inhibiteurs de plus en plus spécifiques de tel ou tel type d'isoenzyme (J. A. BEAVO, Physiological Rev., Vol 75, n° 4, pp 725-749, 1995). Parmi les différentes familles de PDE, la PDE IV a été identifiée dans de très nombreux tissus ou cellules comme le cerveau, le coeur, l'endothélium vasculaire, le muscle lisse vasculaire et trachéobronchique, les cellules hématopoiétiques. L'inhibition des phosphodiestérases ralentit l'hydrolyse des nucléotides cycliques et entraîne une augmentation de la teneur en AMPc et/ou GMPc.

Les inhibiteurs de PDE IV, responsables d'une augmentation des taux d'AMPc, possèdent des activités anti-inflammatoires et des effets relaxants sur le muscle lisse trachéobronchique d'où leur intérêt thérapeutique dans le domaine de la pathologie respiratoire ou des pathologies associées à un processus inflammatoire. (M. N. PALFREYMAN, Drugs of the Future, Vol. 20, n° 8, pp 793-804, 1995; J. P. BARNES, Eur. Respir. J., Vol. 8, pp 457-462, 1995; S. B. CHRISTENSEN et T. J. TORPHY, Annual Reports in Medicinal Chemistry, Vol. 29, pp 185-194, 1994, Academic Press).

Ces composés qui sont des inhibiteurs de phosphodiestérases du groupe IV seront particulièrement intéressants dans les applications thérapeutiques concernant l'inflammation et la relaxation bronchique et plus précisément dans l'asthme et les bronchopathies chroniques obstructives (A. J. DUPLANTIER et J. B. CHENG, Annu. Rep. Med. Chem., Vol. 29, p. 73-81, 1994), (C. D. NICHOLSON et M. SHAHID, Pulmonary Pharmacol., Vol. 7, p. 1-17, 1994), (T. J. TORPHY, G. P. LIVI et S. B. CHRISTENSEN, Drug News Perspect., Vol. 6, p. 03-214, 1993), (J. A. LOWE et J. B. CHENG, Drugs Future, Vol. 17, p. 799-807, 1992), mais aussi dans toutes les affections telles que les rhinites (I. RADERER, E. HAEN, C. SCHUDT et B. PRZYBILLA, Wien. Med. Wochenschr., Vol. 145, p. 456-8, 1995), le syndrome de détresse respiratoire aigu (SDRA) (C. R. TURNER, K. M. ESSER et E.B.WHEELDON, Circulatory Shock, Vol. 39, p. 237-45, 1993), les allergies et les dermatites (J. M. HANIFIN et S. C. CHAN, J. Invest. Dermatol., Vol. 105, p. 84S-88S, 1995), (J. M. HANIFIN, J. Dermatol. Sci., Vol. 1, p. 1-6, 1990), le psoriasis (E. TOUITOU, N. SHACO-EZRA, N. DAYAN, M. JUSHYNSKI, R. RAFAELOFF R. AZOURY, J. Pharm. Sci., Vol. 81, p. 131-4, 1992), (F. LEVI-SCHAFFER et E. TOUITOU, Skin Pharmacol., Vol. 4, p. 286-90, 1991), l'arthrite rhumatoïde (J. M. ANAYA et L. R. ESPINOZA, J. Rheumatol., Vol. 22, p. 595-9, 1995), les maladies autoimmunes, (C. P. GENAIN et al. Proc. Natl. Acad. Sci., Vol. 92, p. 3601-5, 1995), les scléroses multiples (N. SOMMER et al., Nat. Med., Vol. 1, p. 244-8, 1995), les dyskinésies (T. KITATANI, S. HAYASHI et T. SAKAGUCHI, Nippon. Yakurigaku. Zasshi, Vol. 86, p. 353-8, 1985), les glomérulonéphrites (M. HECHT, M. MULLER, M. L. LOHMANN-MATTHES et A. EMMENDORFFER, J. Leukoc. Biol., Vol. 57, p. 242-9, 1995), l'ostéoarthrite et le choc septique (A. M. BADGER, D. L. OLIVERA et K. M. ESSER Circ. Shock, Vol. 44, p. 188-95, 1994; L. SEKUT et al., Clin. Exp. Immunol., Vol. 100, p. 126-32, 1995), le sida (T.F.GRETEN, S. ENDRES et al., Aids, Vol. 9 p.1137-44, 1995), la dépression (N. A. SACCOMANO et al., J. Med. Chem., Vol. 34, p. 291-8, 1991), et toute maladie neurodégénérative s'accompagnant de phénomènes inflammatoires comme les maladies d'Alzheimer, de Parkinson, de Huntington, de Down et la sclérose latérale amyotrophique (G. Z. FEUERSTEIN et al., Ann. N. Y. Acad. Sci., Vol. 765, p. 62-71, 1995).

Ces indications thérapeutiques ne sont pas limitatives dans la mesure où la diminution de la concentration d'AMPc cellulaire, quelqu'en soient la cause et la localisation tissulaire, aboutit à une dysfonction cellulaire, source de phénomènes pathologiques, et peut constituer une cible thérapeutique majeure des produits décrits.

L'état de l'Art le plus proche est notamment représenté par les composés décrits dans le brevet WO 96/03396 qui décrit des dérivés de dihydrobenzofurane en tant qu'agents anti-inflammatoires, et dans le brevet FR 2.142.470 qui décrit des feuilles d'enregistrement contenant un dérivé d'acide carboxylique biaromatique.

Plus spécifiquement, la présente invention concerne les composés de formule (I): 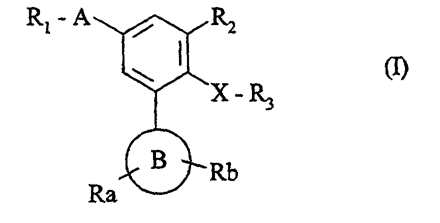 dans laquelle :
- R₁: représente un groupement cycloalkyle (C₃-C₇) substitué ou non, un groupement phényle substitué ou non, un groupement naphtyle substitué ou non, un groupement hétérocyclique, saturé ou insaturé, substitué ou non, mono ou bicyclique contenant de 1 à 3 hétéroatomes choisis parmi azote, oxygène ou soufre, un groupement alkyle (C₁-C₆) linéaire ou ramifié substitué ou non, ou un groupement alkényle (C₁-C₆) linéaire ou ramifié substitué ou non;
- A: représente une liaison (à la condition que dans ce cas R₁ soit différent d'un groupement alkyle C₁ ou C₂), un atome d'oxygène, de soufre, un groupement 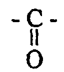 un groupement 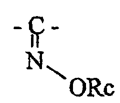 ou un groupement 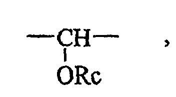 (dans lesquels Rc représente un atome d'hydrogène, un groupement alkyle (C₁-C₆) linéaire ou ramifié ou un groupement cycloalkyle (C₃-C₇)),
- R₂: représente un atome d'halogène, un groupement alkyle (C₁-C₆) linéaire ou ramifié substitué ou non, alkényle (C₁-C₆) linéaire ou ramifié substitué ou non, alkynyle (C₁-C₆) linéaire ou ramifié substitué ou non, cycloalkyle (C₃-C₇) subsitué ou non, formyle, carboxy, alkylcarbonyle (C₁-C₆) linéaire ou ramifié, cycloalkylcarbonyle (C₃-C₇), alkoxycarbonyle (C₁-C₆) linéaire ou ramifié, cycloalkyloxycarbonyle (C₃-C₇), carbamoyle (éventuellement substitué par un ou deux groupements alkyle (C₁-C₆) linéaire ou ramifié, cycloalkyle (C₃-C₇), hydroxy, alkoxy (C₁-C₆) linéaire ou ramifié, cycloalkyloxy (C₃-C₇) ou formant ensemble avec l'atome d'azote qui les porte un cycle de 5 à 7 chaînons contenant de 1 à 3 hétéroatomes choisis parmi azote, oxygène et soufre), amino (éventuellement substitué par un ou deux groupements alkyle (C₁-C₆) linéaire ou ramifié, acyle (C₁-C₆) linéaire ou ramifié, cycloalkyle (C₃-C₇) ou formant ensemble avec l'atome d'azote qui les porte un cycle de 5 à 7 chaînons contenant de 1 à 3 hétéroatomes choisis parmi azote, oxygène et soufre), formamido, cyano, amidino (éventuellement substitué par un ou deux groupements alkyle (C₁-C₆) linéaire ou ramifié, cycloalkyle (C₃-C₇), ou formant ensemble avec l'atome d'azote qui les porte un cycle de 5 à 7 chaînons contenant de 1 à 3 hétéroatomes choisis parmi azote, oxygène et soufre), hydroxyaminométhyle (éventuellement substitué, indépendamment sur l'atome d'azote ou d'oxygène, par un groupement alkyle (C₁-C₆) linéaire ou ramifié, cycloalkyle (C₃-C₇), ou acyle (C₁-C₆) linéaire ou ramifié), amide-oxime (éventuellement substitué, indépendamment sur l'atome d'azote ou d'oxygène par un groupement alkyle (C₁-C₆) linéaire ou ramifié, cycloalkyle (C₃-C₇), ou formant avec l'atome d'azote qui les porte un cycle de 5 à 7 chaînons contenant de 1 à 3 hétéroatomes choisis parmi azote, oxygène et soufre), hydrazono (éventuellement substitué par un ou deux groupements alkyles (C₁-C₆) linéaires ou ramifiés, cycloalkyles (C₃-C₇), ou formant avec l'atome d'azote qui les porte un cycle de 5 à 7 chaînons contenant de 1 à 3 hétéroatomes choisis parmi azote, oxygène et soufre), ou un groupement choisi parmi : 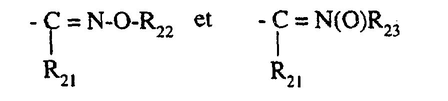 dans lesquels R₂₁ représente un atome d'hydrogène, un groupement alkyle (C₁-C₆) linéaire ou ramifié, ou un groupement cycloalkyle (C₃-C₇), R₂₂ représente un atome d'hydrogène ou un groupement alkyle (C₁-C₆) linéaire ou ramifié, cycloalkyle (C₃-C₇), ou acyle (C₁-C₆) linéaire ou ramifié, R₂₃ représente un groupement alkyle (C₁-C₆) linéaire ou ramifié, ou cycloalkyle (C₃-C₇),
- R₃: représente un atome d'hydrogène, un groupement alkyle (C₁-C₆) linéaire ou ramifié, un groupement cycloalkyle (C₃-C₇) ou un groupement acyle (C₁-C₆) linéaire ou ramifié, ou bien 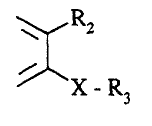 forment ensemble le cycle 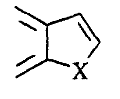
- X: représente un atome d'oxygène, de soufre ou un groupement NR" (dans lequel R" représente un atome d'hydrogène, un groupement alkyle (C₁-C₆) linéaire ou ramifié, cycloalkyle (C₃-C₇) ou acyle (C₁-C₆) linéaire ou ramifié),
le cycle B représente un cycle phényle ou pyridyle,
- Ra, Rb,: identiques ou différents, représentent un atome d'hydrogène, d'halogène, un groupement alkyle (C₁-C₆) linéaire ou ramifié, hydroxy, alkoxy (C₁-C₆) linéaire ou ramifié, carboxy, polyhalogénoalkyle (C₁-C₆) linéaire ou ramifié, cyano, nitro, alkoxycarbonyle (C₁-C₆) linéaire ou ramifié, cycloalkyloxycarbonyle (C₃-C₇), amino (éventuellement substitué par un ou deux groupements alkyle (C₁-C₆) linéaire ou ramifié, acyle (C₁-C₆) linéaire ou ramifié ou cycloalkyle (C₃-C₇)), carbamoyle (éventuellement substitué par un ou deux groupements alkyle (C₁-C₆) linéaire ou ramifié, cycloalkyle (C₃-C₇) ou alkoxy (C₁-C₆) linéaire ou ramifié), sulfo, alkylsulfonyle (C₁-C₆) linéaire ou ramifié, cycloalkylsulfonyle (C₃-C₇) ou aminosulfonyle (éventuellement substitué par un ou deux groupements alkyle (C₁-C₆) linéaire ou ramifié ou cycloalkyle (C₃-C₇),
étant entendu que
- lorsque A représente une liaison ou un atome d'oxygène, B représente un cycle phényle, R₁ représente un groupement cycloalkyle (C₃-C₇) substitué ou non, un groupement phényle substitué ou non, un groupement naphtyle substitué ou non, X représente un atome d'oxygène ou un groupement NR" (dans lequel R" représente un atome d'hydrogène ou un groupement alkyle (C₁-C₆) linéaire ou ramifié) et R₃ représente un atome d'hydrogène ou un groupement alkyle (C₁-C₆) linéaire ou ramifié, alors R₂ est différent d'un groupement carboxy,
leurs énantiomères, diastéréoisomères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthanesulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Parmi les composés préférés de la présente invention, on peut citer les composés tels que X représente un atome d'oxygène, ceux dans lesquels B représente un cycle phényle ou ceux tels que R₁ représente un cycle phényle substitué ou non, ou pyridyle substitué ou non.

Parmi les groupements hétérocycliques saturés ou insaturés, mono ou bicycliques contenant de 1 à 3 hétéroatomes choisis parmi azote, oxygène ou soufre, on peut citer à titre préférentiel les groupements furyle, thiényle, pyridyle ou imidazolyle.

Par groupement alkyle substitué, alkényle substitué, alkynyle substitué ou cycloalkyle substitué, on entend substitué par un ou plusieurs atomes d'halogènes ou groupements alkoxy (C₁-C₆) linéaire ou ramifié, alkylthio (C₁-C₆) linéaire ou ramifié, cycloalkyle (C₃-C₇), phényle substitué ou non, naphtyle substitué ou non ou hétérocyclique substitué ou non (comme par exemple un groupement pyridyle). Un groupement alkyle saturé ou insaturé substitué préféré est le groupement benzyle substitué ou non.

Par groupement phényle substitué, naphtyle substitué ou hétérocyclique substitué ou benzyle substitué, on entend substitué par un ou plusieurs atomes d'halogène ou groupements alkyle (C₁-C₆) linéaire ou ramifié, alkoxy (C₁-C₆) linéaire ou ramifié, ou trihalogénométhyle.

La présente invention s'étend également au procédé de préparation des composés de formule (I), caractérisé en ce que l'on utilise comme produit de départ un composé de formule (II) : 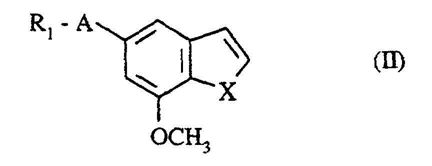 dans laquelle R₁, A et X ont la même signification que dans la formule (I),
que l'on soumet à l'action du tribromure de bore en milieu dichlorométhane, pour conduire au composé de formule (III) : 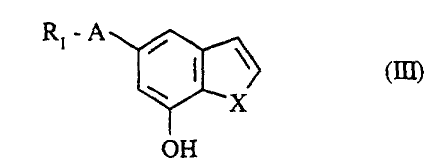 dans laquelle R₁, A et X ont la même signification que dans la formule (I),
que l'on soumet à l'action de l'anhydride trifluorométhanesulfonique dans de la pyridine, pour conduire au composé de formule (IV) : 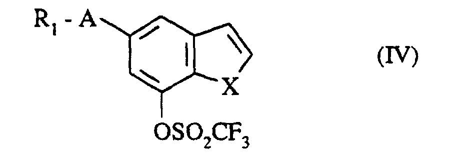 dans laquelle R₁, A et X ont la même signification que dans la formule (I),
qui subit l'action d'un acide borique de formule (V) : 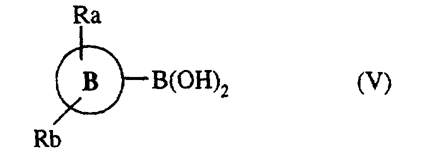 dans laquelle B, Ra et Rb sont tels que définis dans la formule (I),
pour conduire au composé de formule (I/a), cas particulier des composés de formule (I) : 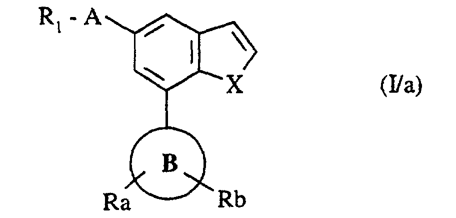 dans laquelle R₁, A, Ra, Rb, B et X ont la même signification que dans la formule (I), composé de formule (I/a), qui subit, si on le désire, une coupure oxydante, pour conduire au composé de formule (I/b), cas particulier des composés de formule (I): 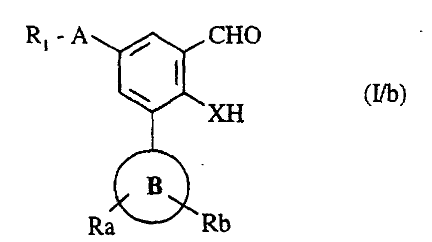 dans laquelle R₁, A, Ra, Rb, B et X ont la même signification que dans la formule (I),
composé de formule (I/b) qui peut subir :
**a** soit l'action du composé de formule (VI) en milieu basique :

   R₂₂O - NH₂ (VI)

   dans laquelle R₂₂ représente un atome d'hydrogène ou un groupement alkyle (C₁-C₆) linéaire ou ramifié, cycloalkyle (C₃-C₇) ou acyle (C₁-C₆) linéaire ou ramifié,
   pour conduire au composé de formule( I/c), cas particulier des composés de formule (I) : 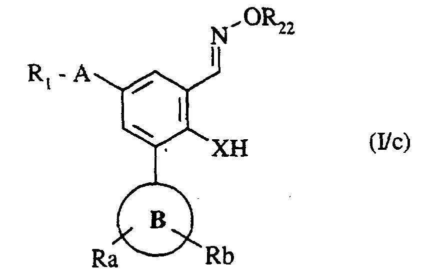 dans laquelle R₁, A, Ra, Rb, B, X et R₂₂ sont tels que définis précédemment,
   composé de formule (I/c), que l'on transforme, éventuellement, lorsque R₂₂ est différent d'un groupement acyle, en milieu acide, en composé de formule (I/d), cas particulier des composés de formule (I): 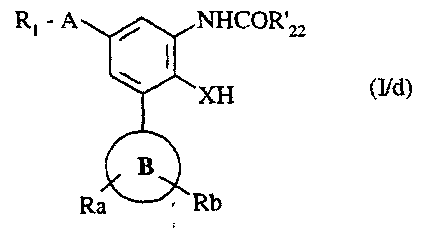 dans laquelle R₁, A, Ra, Rb, B et X ont la même signification que précédemment et R'₂₂ différent d'un groupement acyle à la même définition que R₂₂,
**b** soit l'action, en milieu alcoolique, d'une hydroxylamine de formule R₂₃NH - OH dans laquelle R₂₃ représente un groupement alkyle (C₁-C₆) linéaire ou ramifié, ou cycloalkyle (C₃-C₇),
   pour conduire au composé de formule (I/e), cas particulier des composés de formule (I) : 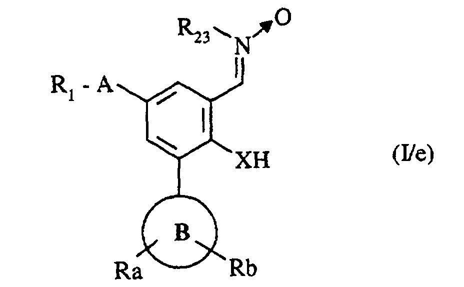 dans laquelle R₁, A, B, Ra, Rb, X et R₂₃ ont la même signification que précédemment,
**c** soit l'action d'un ylure de phosphore de formule R'cCH₂P⁺(C₆H₅)₃Br⁻ (dans laquelle R'c représente un atome d'hydrogène, un groupement alkyle (C₁-C₆) linéaire ou ramifié, cycloalkyle (C₃-C₇), alkoxy (C₁-C₆) linéaire ou ramifié, alkylthio (C₁-C₆) linéaire ou ramifié, phényle substitué ou non, naphtyle substitué ou non, hétérocyclique saturé ou insaturé substitué ou non), en présence de n-butyllithium,
   pour conduire au composé de formule (I/f), cas particulier des composés de formule (I) : 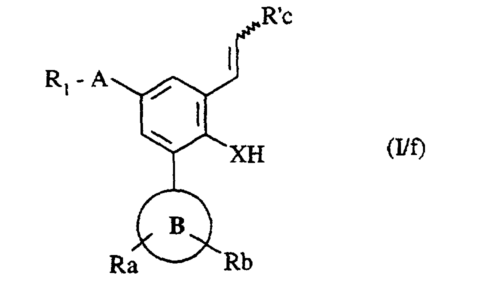 dans laquelle R₁, A, Ra, Rb, B, X et R'c ont la même signification que précédemment, qui subit, si on le souhaite, une réduction, pour conduire au composé de formule (I/g), cas particulier des composés de formule (I) : 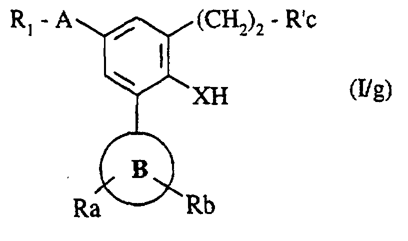 dans laquelle R₁, A, Ra, Rb, B, X et R'c ont la même signification que précédemment,
**d** soit une réaction en milieu oxydant, pour conduire au composé de formule (I/h) : 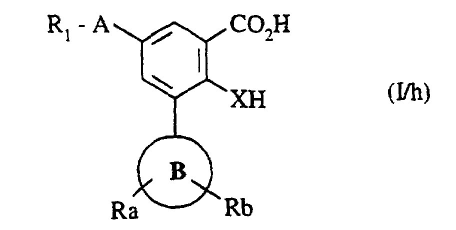 dans laquelle R₁, A, Ra, Rb, B et X ont la même signification que dans la formule (I), composé de formule (I/h) dont on transforme la fonction acide en chlorure d'acide correspondant, qui peut alors être transformé en ester, en amide ou en amine correspondants selon les réactions classiques de la chimie organique,
**e** soit, l'action du chlorhydrate d'hydroxylamine en milieu approprié, pour conduire au composé de formule (I/i), cas particulier des composés de formule (I) : 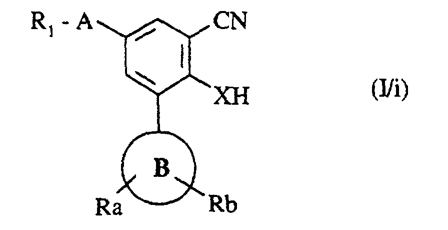 dans laquelle R₁, A, Ra, Rb, B et X ont la même signification que précédemment,
   - que l'on fait réagir en milieu alcoolique avec de l'acide chlorhydrique, puis avec une amine Rd-NH-Re (dans laquelle Rd, Re, identiques ou différents, représentent un atome d'hydrogène ou un groupement alkyle (C₁-C₆) linéaire ou ramifié, ou cycloalkyle ou formant ensemble avec l'atome d'azote qui les porte un cycle de 5 à 7 chaînons contenant de 1 à 3 hétéroatomes choisis parmi oxygène, azote et soufre),
pour conduire au composé de formule (I/j), cas particulier des composés de formule (I): 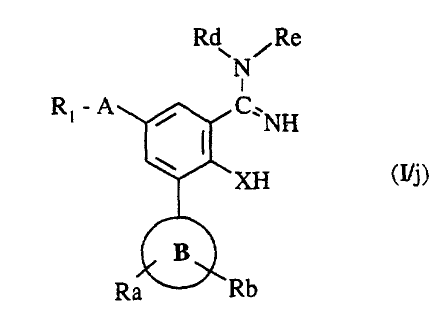 dans laquelle R₁, A, Ra, Rb, X, B, Rd et Re sont tels que définis précédemment,
- ou que l'on fait réagir avec un composé de formule (VI) en milieu basique :

   RcO-NH₂ (VI)

   dans laquelle Rc est tel que défini dans la formule (I),
pour conduire au composé de formule (I/k), cas particulier des composés de formule (I): 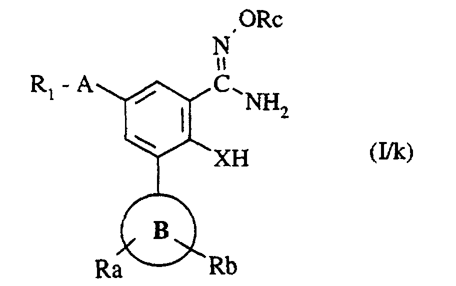 dans laquelle A, B, R₁, Ra, Rb, Rc, et X sont tels que définis précédemment, **f** soit, enfin avec une hydrazine 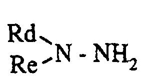 (Rd et Re étant tels que définis précédemment), pour conduire au composé de formule (I/l), cas particulier des composés de formule (I): 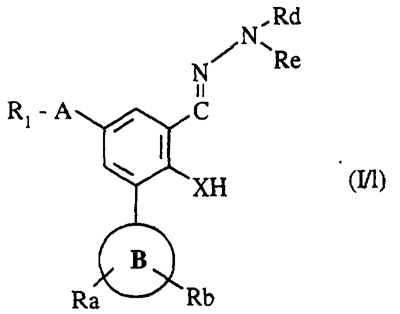 dans laquelle A, B, R₁, Ra, Rb, Rd, Re et X sont tels que définis précédemment,
composé de formule (I/a), (I/b), (I/c), (I/d), (I/e), (I/f), (I/g), (I/h), (I/i), (I/l)ou leurs dérivés :
- que l'on soumet éventuellement à l'action d'un dialkylsulfate ou d'un halogénure d'alkyle (C₁-C₆) linéaire ou ramifié ou d'acyle (C₁-C₆) linéaire ou ramifié ou de cycloalkyle (C₃-C₇), pour conduire au composé de formule (I/m), cas particulier des composés de formule (I): 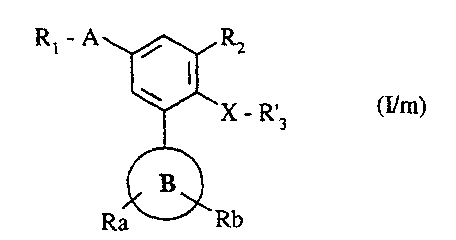 dans laquelle R₁, A, B, Ra, Rb, R₂ et X ont la même signification que précédemment, et R'₃ représente un groupement alkyle (C₁-C₆) linéaire ou ramifié, acyle (C₁-C₆) linéaire ou ramifié ou cycloalkyle (C₃-C₇),
composé de formule (I/a) à (I/m) ou leurs dérivés :
- qui peut être, le cas échéant, purifié selon une technique classique de purification
- dont on sépare, le cas échéant, les isomères selon une technique classique de séparation,
- que l'on transforme, si on le souhaite, en ses sels d'addition à un acide ou à une base pharmaceutiquement acceptable.

Les composés de formule (II) sont généralement préparés à partir d'un aldéhyde de formule (VII) : 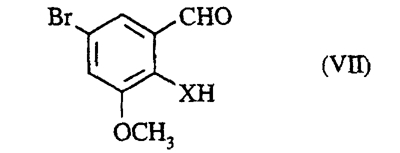 dans laquelle X a la même signification que dans la formule (I),
que l'on fait réagir avec le 2-bromo-1,1-diméthoxyéthane en milieu approprié, pour conduire au composé de formule (VIII) : 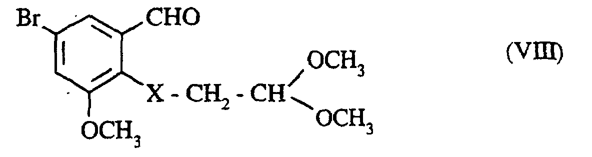 dans laquelle X a la même signification que dans la formule (I),
que l'on cyclise en milieu acide, pour conduire au composé de formule (IX): 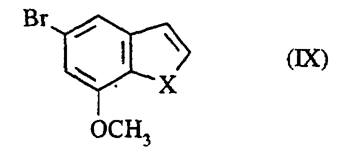 dans laquelle X a la même signification que dans la formule (I),
composé de formule (IX), qui subit :
- soit, l'action d'un organozincique de formule R₁ - CH₂ - ZnBr (dans laquelle R₁ a la même signification que dans la formule (I),
- soit, après réaction préalable avec de l'hexabutyldiétain, l'action d'un chlorure d'acide de formule R₁ - CO - Cl (dans laquelle R₁ a la même signification que dans la formule (I), en présence d'un catalyseur palladié,
étant entendu que la fonction carbonyle peut éventuellement être réduite en hydroxyle par les réducteurs classiquement utilisés en chimie organique, lorsque cela est compatible avec les substituants présents sur la molécule,
- soit, après réaction préalable d'un dérivé éthylénique de formule 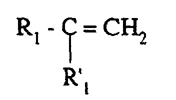 (dans laquelle R₁ a la même signification que dans la formule (I) et R'₁ représente un atome d'hydrogène ou un groupement alkyle (C₁-C₆) linaire ou ramifié, cycloalkyle (C₃-C₇)) avec le 9-borabicyclo[3.3.1]nonane, puis réaction en présence d'un catalyseur palladié,
- soit, en présence d'un catalyseur palladié, l'action d'un composé de formule (X) :

   R₁-YH (X)

   dans laquelle R₁ a la même signification que dans la formule (I) et Y représente un atome de soufre ou d'oxygène,
   pour conduire au composé de formule (II).

Certains composés de formule (I) peuvent avantageusement être obtenus en utilisant comme produit de départ, lorsqu'il est disponible, un composé de formule (XI) : 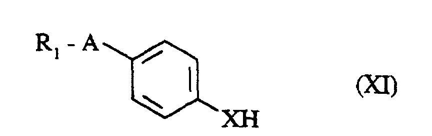 dans laquelle X, A et R₁ ont la même signification que dans la formule (I),
qui subit une réaction de bromation pour conduire au composé de formule (XII) 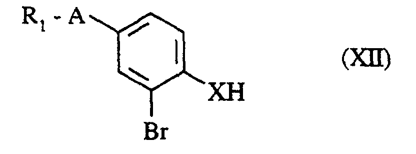 dans laquelle X, A et R₁ ont la même signification que précédemment,
qui est soumis à l'action d'un acide borique de formule (V) : 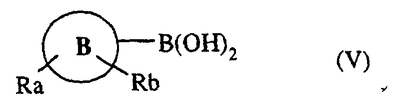 dans laquelle Ra, Rb, et B sont tels que définis dans la formule (I), pour conduire à un composé de formule (XIII): 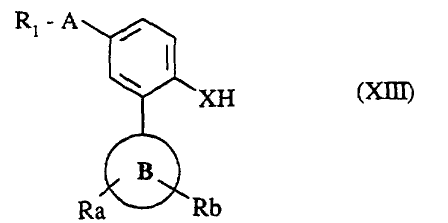 dans laquelle A, R₁, Ra, Rb, B et X sont tels que définis précédemment,
composé (XIII) qui subit une réaction d'halogénation pour conduire au composé de formule (I/n), cas particulier des composés de formule (I): 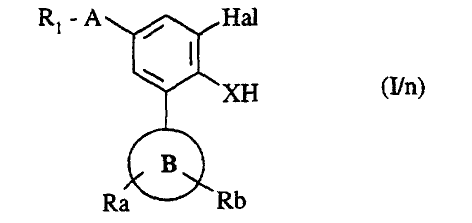 dans laquelle A, R₁, Ra, Rb, B et X sont tels que définis précédemment et Hal représente un halogène,
composé (I/n) qui peut être soumis :
- soit à l'action du cyanure de zinc en présence d'un catalyseur palladié, pour conduire à un composé de formule (I/i) telle que définie précédemment,
- soit à l'action d'un dérivé de l'étain en présence d'un catalyseur palladié, pour conduire à un composé de formule (I/p), cas particulier des composés de formule (I): 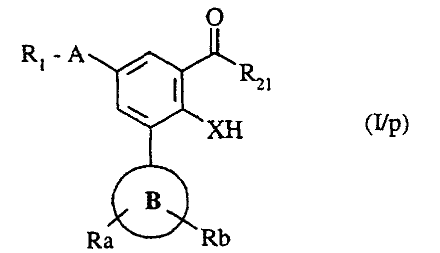 dans laquelle A, R₁, Ra, Rb, B et X sont tels que définis dans la formule (I) et R₂₁, différent d'un atome d'hydrogène a la même définition que dans la formule (I), composé (I/p) que l'on fait réagir :

- soit avec un composé de formule (VI):

   R₂₂O-NH₂ (VI)

   dans laquelle R₂₂ est tel que défini précédemment,
pour conduire au composé de formule (I/q), cas particulier des composés de formule (I) : 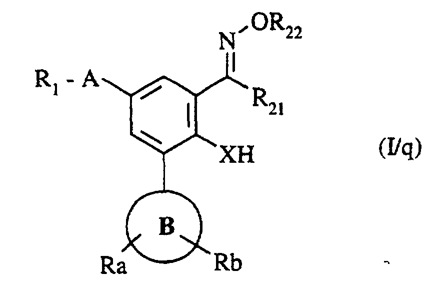 dans laquelle A, B, R₁, Ra, Rb, R₂₁, R₂₂ et X sont tels que définis précédemment ;
- soit avec une hydrazine 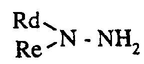 (Rd et Re étant tels que définis précédemment), pour conduire au composé de formule (I/s), cas particulier des composés de formule (I) : 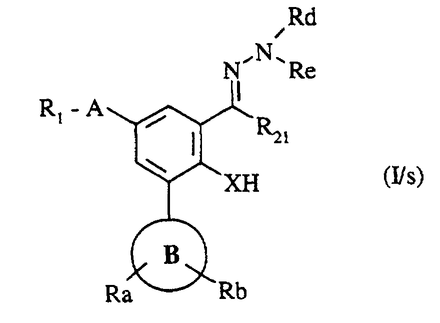 dans laquelle A, B, R₁, Ra, Rb, Rd, Re, R₂₁, et X sont tels que définis précédemment,
   composés (I/n), (I/p), (I/q), (I/s) ou leurs dérivés que l'on soumet éventuellement à l'action d'un diallkylsulfate ou d'un halogénure d'alkyle (C₁-C₆) linéaire ou ramifié, ou d'acyle (C₁-C₆) linéaire ou ramifié, ou de cycloalkyle (C₃-C₇), pour conduire au composé de formule (I/m) telle que définie précédemment,
composés (I/n) à (I/s) ou leur dérivés :
- qui peuvent être, le cas échéant, purifiés selon une technique classique de purifiaction,
- dont on sépare éventuellement les isomères selon une technique classique de séparation,
- que l'on transforme, si on le souhaite, en leurs sels d'addition à un acide ou à une base pharmaceutiquement acceptable.

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, les aérosols, etc... La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 10 et 5000 mg pour un traitement en 1 à 3 prises par 24 heures.

Les exemples suivants illustrent l'invention et ne la limitent en aucune façon.

Les produits de départ utilisés sont des produits connus ou préparés selon des modes opératoires connus.

Les structures des composés décrits dans les exemples ont été déterminées selon les techniques spectrophotométriques usuelles (infrarouge, RMN, spectrométrie de masse ...).

### EXEMPLE 1 : 5-Benzyl-7-(3-nitrophényl)benzo[b]furane

### Stade A : 5-Bromo-2-(2,2-diméthoxyéthoxy)-3-méthoxybenzaldéhyde

Une solution hétérogène de 31 g (0,134 mol) de 5-bromo-2-hydroxy-3-méthoxybenzaldéhyde, 28 g (0,166 mol) de 2-bromo-1,1-diméthoxyéthane et de 49 g (0,150 mol) de carbonate de césium dans 150 ml de diméthylformamide est portée à 160°C pendant 3 heures. Après retour à température ambiante, le mélange est filtré. Le filtrat est ensuite évaporé sous pression réduite. Le solide ainsi obtenu est repris par 250 ml de dichlorométhane et à nouveau filtré. La solution homogène est alors lavée par deux fois 200 ml d'acide chlorhydrique 0,1 N puis par 200 ml d'une solution de chlorure de sodium saturée. La phase organique est ensuite séchée sur sulfate de sodium puis évaporée sous pression réduite et conduit au produit attendu.

### Stade B : 5-Bromo-7-méthoxybenzo[b]furane

Le produit obtenu au stade précédent est solubilisé dans 1000 ml d'acide sulfurique à 20% et porté à reflux pendant 3 heures. Après refroidissement à température ambiante, la solution est neutralisée au carbonate de potassium puis extraite par deux fois 300 ml d'acétate d'éthyle. Les phase organiques sont ensuite réunies, séchées sur sulfate de sodium, filtrées puis concentrées sous pression réduite.
Le résidu est purifié par chromatographie sur colonne de silice en utilisant comme éluant un mélange dichlorométhane / heptane (1/1) et conduit au produit attendu sous forme d'huile incolore qui cristallise au froid.

### Stade C : 5-Benzyl-7-méthoxybenzo[b]furane

12,3 g (0,188 At-g) de zinc en poudre sont recouverts par 15 ml de tétrahydrofurane auxquels on ajoute quelques gouttes de dibromoéthane et le tout est placé sous atmosphère d'argon. On chauffe rapidement la solution et coule quelques gouttes d'une solution de 29,4 g (0,171 mol) de bromure de benzyle dans 135 ml de tetrahydrofurane. Lorsque la réaction est amorcée, le mélange réactionnel est refroidi à + 4°C, et l'addition du restant de bromure de benzyle en solution s'opère à cette température (temps d'addition environ 1 heure). Le mélange est ensuite agité à + 4°C pendant 2 à 3 heures.
Cette solution est ensuite additionnée goutte à goutte à un mélange de 19,5 g (0,086 mol) du composé obtenu au stade précédent et de 5,18 g (4,47.10⁻³ mol) de tétrakistriphényl phosphine Palladium (O) dans 150 ml de tétrahydrofurane sous atmosphère d'argon. Le mélange réactionnel est porté à reflux pendant 15 heures. Après retour à température ambiante, le milieu réactionnel est dilué par 300 ml d'acétate d'ethyle puis lavé successivement par 200 ml d'eau et 200 ml d'une solution saturée de chlorure de sodium. Le traitement habituel de la phase organique livre une huile qui est purifiée par chromatographie sur colonne de silice en utilisant comme éluant un mélange dichlorométhane / heptane (1/1) et conduit au produit attendu sous forme d'huile.

### Stade D : 5-Benzyl-7-hydroxybenzo[b]furane

Une solution de 18 g (0,0755 mol) du composé obtenu au stade précédent et de 150 ml de dichlorométhane est additionnée, sous atmosphère d'argon, à une solution de 155 ml de tribromure de bore 1 M dans le dichlorométhane à -78°C. Le mélange réactionnel est graduellement remonté jusqu'à une température de -5°C (environ 15 heures). A cette température, 150 ml d'eau sont coulés et la phase organique est séparée. La phase aqueuse est saturée avec du chlorure de sodium et réextraite à l'aide de 100 ml de dichlorométhane. Les phases organiques sont alors réunies, séchées sur sulfate de sodium, filtrées et concentrées sous pression réduite. Le résidu est purifié par chromatographie sur colonne de silice en utilisant comme éluant le dichlorométhane et conduit au produit attendu sous forme d'huile.

### Stade E : 5-Benzyl-7-trifluorométhanesulfonyloxybenzo[b]furane

Sous atmosphère inerte et à 0°C, 18,3 g (0,0649 mol) d'anhydride trifluorométhanesulfonique sont coulés goutte à goutte à une solution de 9,1 g (0,04057 mol) du composé obtenu au stade précédent dans 60 ml de pyridine. Après 2 heures, la solution est concentrée sous pression réduite. Le résidu est repris à l'aide de 100 ml de dichlorométhane et lavé successivement par 150 ml d'une solution d'acide chlorhydrique 2 N, 150 ml d'une solution de bicarbonate de sodium saturée et enfin 150 ml d'une solution de chlorure de sodium saturée. Le traitement habituel de la phase organique conduit au produit attendu sous forme semi-solide.

### Stade F : 5-Benzyl-7-(3-nitrophényl)benzo[b]furane

Sous atmosphère inerte, un mélange de 11,1 g (0,08 mol) de carbonate de potassium, 13 g (0,0364 mol) du composé décrit au stade précedent, 8,6 g (0,05 mol) d'acide 3-nitrophénylborique et de 1,49 g (1,82.10⁻³ mol) chlorure de bis (diphénylphosphino)férrocène palladium (II) dans 250 ml de diméthylformamide est porté à 80°C pendant 18 heures. Après concentration sous pression réduite du solvant, le résidu est repris par 200 ml d'acétate d'éthyle et lavé par 200 ml d'eau puis 200 ml d'une solution saturée de chlorure de sodium. Un traitement habituel de la phase organique suivi d'une purification par chromatographie sur colonne de silice (éluant : toluène - heptane 6/4 puis 8/1) permet d'obtenir 10,11 g (0,0307 mol) d'une huile jaune pâle.

| *Microanalyse élémentaire :* | | | |
|---|---|---|---|
| | *C %* | *H %* | *N %* |
| *calculé* | *77,07* | *4,82* | *4,13* |
| *trouvé* | *76,58* | *4,59* | *4,25* |

### EXEMPLE 2 : 5-(4-Fluorobenzyl)-7-(3-nitrophényl)benzo[b]furane

Les stades A et B sont identiques aux stades A et B de l'exemple 1.

### Stade C : 5-(4-Fluorobenzyl)-7-méthoxybenzo[b]furane

Le produit attendu est obtenu selon le procédé décrit dans l'exemple 1 en remplaçant au stade C le bromure de benzyle par le bromure de 4-fluorobenzyle.
*Point de fusion : 76-78°C*

Les stades D, E et F sont identiques aux stades D, E et F de l'exemple 1.

### Stade D : 5-(4-Fluorobenzyl)-7-hydroxybenzo[b]furane

### Stade E : 5-(4-Fluorobenzyl)-7-trifluorométhanesulfonyloxybenzo[b]furane

### Stade F : 5-(4-Fluorobenzyl)-7-(3-nitrophényl)benzo[b]furane

| *Microanalyse élémentaire :* | | | |
|---|---|---|---|
| | *C%* | *H%* | *N%* |
| *calculé* | *72,62* | *4,06* | *4,03* |
| *trouvé* | *72,47* | *4,14* | *4,11* |

### EXEMPLE 3 : 5-(2,6-Dichloro-4-pyridylméthyl)-7-(3-nitrophényl)benzo[b]furane

Le produit attendu est obtenu selon le procédé décrit dans l'exemple 1 en remplaçant au stade C le bromure de benzyle par le bromure de 2,6-dichloro-4-pyridylméthyle.

### EXEMPLE 4 : 5-(2-Furoyl)-7-(3-nitrophényl)benzo[b]furane

### Stade A : 5-Tributylstannyl-7-méthoxybenzo[b]furane

Une solution de 10 g (0,044 mol) de 5-bromo-7-méthoxybenzo[b]furane décrit au stade B de l'exemple 1, 51,10 g (0,088 mol) d'hexabutyldiétain et de 2,55 g (0,0022 mol) de tétrakistriphénylphosphine Palladium (O) dans 200 ml de toluène est chauffée à 100°C, sous atmosphère d'argon pendant 4 heures. Après retour à l'ambiante, la solution est agitée toute la nuit avec une solution de 80 ml de fluorure de potassium méthanolique. Le mélange hétérogène est alors filtré sur célite. Le méthanol est ensuite évaporé et la solution toluénique restante est lavée à l'aide de 100 ml d'eau puis 100 ml d'une solution de chlorure de sodium saturée. Le traitement habituel de la phase organique livre une huile qui est purifiée par chromatographie sur colonne de silice en utilisant comme éluant un mélange toluène / heptane (4/6) et conduit au produit attendu sous forme d'huile.

### Stade B : 5-(2-Furoyl)-7-méthoxybenzo[b]furane

Une solution de 5 g (0,0114 mol) du dérivé de l'étain décrit au stade précédent, 1,49 g (0,0114 mol) de chlorure de 2-furoyle et de 0,12 g (0,000134 mol) de tris(dibenzylidèneacétone) dipalladium (O) dans 50 ml de toluène est portée à 75-80°C pendant 1heure 30. Après retour à température ambiante, la solution est filtrée sur célite puis lavée par 50 ml d'eau puis 50 ml d'une solution saturée de chlorure de sodium. Le traitement habituel de la phase organique livre une huile qui est purifiée par chromatographie sur colonne de silice en utilisant comme éluant le dichlorométhane.
*Point de fusion : 121-123°C*

### Stade C : 5-(2-Furoyl)-7-hydroxybenzo[b]furane

Le produit attendu est obtenu selon le procédé décrit au stade D de l'exemple 1 à partir du composé obtenu au stade précédent.
*Point de fusion : 140-142°C*

### Stade D : 5-(2-Furoyl)-7-trifluorométhanesulfonyloxybenzo[b]furane

Le produit est obtenu selon le procédé décrit au stade E de l'exemple 1 à partir du composé obtenu au stade précédent.

### Stade E : 5-(2-Furoyl)-7-(3-nitrophényl)benzo[b]furane

Le produit attendu est obtenu selon le procédé décrit au stade F de l'exemple 1 à partir du composé obtenu au stade précédent.
*Point de fusion : 148-150°C*

| *Microanalyse élémentaire :* | | | |
|---|---|---|---|
| | *C %* | *H %* | *N %* |
| *calculé* | *68,47* | *3,33* | *4,20* |
| *trouvé* | *68,42* | *3,56* | *4,48* |

### EXEMPLE 5 : 7-(3-Nitrophényl)-5-pentylbenzo[b]furane

### Stade A : 7-Méthoxy-5-pentylbenzo[b]furane

Sous atmosphère inerte et à 0°C, une solution de 58,2 ml de 9-BBN 0,5 molaire est additionnée goutte à goutte à une solution de 2,04 g (0,029 mol) de pentène dans 12 ml tétrahydrofurane. En fin d'addition, le bain de glace est enlevé et la solution est agitée pendant 3 heures. Cette solution est ensuite coulée goutte à goutte à une solution de 6 g (0,0264 mol) de 5-bromo-7-méthoxybenzo[b]furane, 0,65 g (0,000796 mol) de complexe de palladium décrit au stade F de l'exemple 1, 24 ml de soude 3 N dans 84 ml de tétrahydrofurane. Le mélange réactionnel est chauffé à 65°C pendant 15 heures. Après retour à température ambiante, la solution est diluée par 150 ml d'acétate d'éthyle et lavée à l'eau puis par 150 ml d'une solution d'acide chlorhydrique 0,1 N et enfin 100 ml d'une solution de chlorure de sodium saturée. Le traitement habituel de la phase organique donne une huile qui est purifiée par chromatographie sur colonne de silice en utilisant comme éluant un mélange toluène / cyclohexane (4/6).

### Stade B : 7-Hydroxy-5-pentylbenzo[b]furane

Le produit attendu est obtenu selon le procédé décrit au stade D de l'exemple 1 à partir du composé obtenu au stade précédent.

### Stade C : 5-Pentyl-7-trifluorométhanesulfonyloxybenzo[b]furane

Le produit est obtenu selon le procédé décrit au stade E de l'exemple 1 à partir du composé obtenu au stade précédent.

### Stade D : 7-(3-Nitrophényl)-5-pentylbenzo[b]furane

Le produit attendu est obtenu selon le procédé décrit au stade F de l'exemple 1 à partir du composé obtenu au stade précédent.

| *Microanalyse élémentaire :* | | | |
|---|---|---|---|
| | *C %* | *H %* | *N %* |
| *calculé* | *73,77* | *6,19* | *4,53* |
| *trouvé* | *74,43* | *6,28* | *4,65* |

### EXEMPLES 6 et 7 :

### EXEMPLE 6 : 5-Allyl-7-(3-nitrophényl)benzo[b]furane

### EXEMPLE 7 : (E)-7-(3-Nitrophényl)-5-(prop-1-ényl)benzo[b]furane

### Stade A : 5-Allyl-7-méthoxybenzo[b]furane

Une solution de 10 g (0,044 mol) de 5-bromo-7-méthoxybenzo[b]furane, 17,5 g (0,053 mol) d'allyltributylétain et de 1,02 g (0,00088 mol) de tétrakistriphénylphosphine Palladium (O) dans 80 ml de diméthylformamide est chauffée à 90°C pendant 15 heures sous atmosphère inerte. Après retour à température ambiante, la diméthylformamide est évaporée sous vide. La masse réactionnelle est reprise par 200 ml d'éther diéthylique et lavée successivement par 100 ml d'eau et 100 ml d'une solution de chlorure de sodium saturée. Le traitement habituel de la phase organique donne une huile qui est purifiée par chromatographie sur colonne de silice en utilisant comme éluant un mélange dichlorométhane / heptane (6/4).

### Stade B : Mélange de 5-Allyl-7-trifluorométhanesulfonyloxybenzo[b]furane (B₁) et de (R,S)-5-(2-Bromoprop-1-yl)-7-trifluorométhanesulfonyloxybenzo[b]furane (B₂)

Le mélange de ces deux composés est obtenu en réalisant successivement les procédés décrits aux stades D et E de l'exemple 1 à partir du composé décrit au stade précédent. Les composés B₁ et B₂ sont séparés par chromatographie sur colonne de silice en utilisant comme éluant un mélange toluène / heptane (2/8).

### 5-Allyl-7-(3-nitrophényl)benzo[b]furane

Le produit attendu est obtenu selon le procédé décrit au stade F de l'exemple 1 à partir du composé B₁ obtenu au stade précédent.

| *Microanalyse élémentaire :* | | | |
|---|---|---|---|
| | *C %* | *H %* | *N %* |
| *calculé* | *72,11* | *4,69* | *5,01* |
| *trouvé* | *72,60* | *4,93* | *5,07* |

### (E)-7-(3-Nitrophényl)-5-(prop-1-ényl)benzo[b]furane

Le produit attendu est obtenu selon le procédé décrit au stade F de l'exemple 1 à partir du composé B₂ obtenu au stade précédent.
*Point de fusion : 103-105°C*

| *Microanalyse élémentaire :* | | | |
|---|---|---|---|
| | *C %* | *H %* | *N %* |
| *calculé* | *72,11* | *4,69* | *5,01* |
| *trouvé* | *72,31* | *4,95* | *4,99* |

### EXEMPLE 8 : 5-Benzyl-2-hydroxy-3-(3-nitrophényl)benzaldéhyde

Un bullage d'ozone est pratiqué dans une solution de 3,4 g (0,01 mol) du composé décrit dans l'exemple 1 dans 150 ml de dichlorométhane colorée au «Sudan Red» à -78°C. La décoloration (3 heures) signifie la fin de réaction. On ajoute alors 4,65 g (0,075 mol) de diméthylsulfure et la solution est agitée toute la nuit à température ambiante. La solution est ensuite lavée par 50 ml d'eau puis 50 ml d'une solution de chlorure de sodium saturée. Le traitement habituel de la phase organique suivi d'une purification par chromatographie sur colonne de silice en utilisant comme éluant un mélange dichlorométhane / heptane (8/2) donne le produit attendu sous forme d'huile incolore.

| *Microanalyse élémentaire :* | | | |
|---|---|---|---|
| | *C %* | *H %* | *N %* |
| *calculé* | *72,06* | *4,54* | *4,20* |
| *trouvé* | *71,17* | *4,71* | *4,28* |

### EXEMPLE 9 : 5-(4-Fluorobenzyl)-2-hydroxy-3-(3-nitrophényl)benzaldéhyde

Le produit attendu est obtenu selon le procédé décrit dans l'exemple 8 à partir du composé décrit dans l'exemgle 2.

### EXEMPLE 10 : 5-(2,6-Dichloro-4-pyridylméthyl)-2-hydroxy-3-(3-nitrophényl)benzaldéhyde

Le produit attendu est obtenu selon le procédé décrit dans l'exemple 8 à partir du composé décrit dans l'exemple 3.

### EXEMPLE 11 : 5-Benzyl-7-(4-pyridyl)benzo[b]furane

Le produit attendu est obtenu selon le procédé décrit dans l'exemple 1 en remplaçant au stade F l'acide 3-nitrophénylborique par l'acide 4-pyridylborique.

### EXEMPLE 12 : 2-Hydroxy-3-(3-nitrophényl)-5-pentylbenzaldéhyde

Le produit attendu est obtenu selon le procédé décrit dans l'exemple 8 à partir du composé décrit dans l'exemple 5.

### EXEMPLE 13 : 5-Benzyl-7-(3-nitrophényl)indole

Le produit attendu est obtenu selon le procédé décrit dans l'exemple 1 en remplaçant au stade A le 5-bromo-2-hydroxy-3-méthoxybenzaldéhyde par le 2-amino-5-bromo-3-méthoxy benzaldéhyde.
*Point de fusion : 155-157 °C*

| *Microanalyse élémentaire :* | | | |
|---|---|---|---|
| | *C %* | *H %* | *N %* |
| *calculé* | *76,81* | *4,91* | *8,53* |
| *trouvé* | *76,97* | *4,86* | *8,38* |

### EXEMPLE 14 : 2-Amino-5-benzyl-3-(3-nitrophényl)benzaldéhyde

Le produit attendu est obtenu selon le procédé décrit dans l'exemple 8 à partir du composé décrit dans l'exemple 13.

### EXEMPLE 15 : (E)-5-Benzyl-2-hydroxy-3-(3-nitrophényl)benzaldéhyde oxime

Une solution de 0,9 g (0,0027 mol) du composé décrit dans l'exemple 8 et de 0,2 g (0,0028 mol) de chlorhydrate d'hydroxylamine dans 8 ml de pyridine est chauffée à reflux pendant 1heure 30. Le solvant est évaporé et le solide est repris par 30 ml de dichlorométhane. La solution est ensuite lavée par 10 ml d'une solution d'acide chlorhydrique 0,5 N puis 10 ml d'une solution de chlorure de sodium saturée. Le traitement habituel de la phase organique donne le produit attendu sous forme solide.
*Point de fusion : 143-145°C (éther de pétrole).*

### EXEMPLE 16 : (E)-5-(4-Fluorobenzyl)-2-hydroxy-3-(3-nitrophényl)benzaldéhyde oxime

Le produit attendu est obtenu selon le procédé décrit dans l'exemple 15 à partir du composé décrit dans l'exemple 9.
*Point de fusion : 157-159°C (toluène)*

| *Microanalyse élémentaire :* | | | |
|---|---|---|---|
| | *C %* | *H %* | *N %* |
| *calculé* | *65,57* | *4,13* | *7,65* |
| *trouvé* | *65,35* | *4,43* | *7,63* |

### EXEMPLE 17 : (E)-2-Hydroxy-3-(3-nitrophényl)-5-pentylbenzaldéhyde oxime

Le produit attendu est obtenu selon le procédé décrit dans l'exemple 15 à partir du composé décrit dans l'exemple 12.
*Point de fusion : 106-108°C (éthanol-eau)*

### EXEMPLE 18 : 3-Benzyl-6-méthoxy-5-(3-nitrophényl)benzaldéhyde

Une solution de 0,2 g (0,0006 mol) du composé décrit dans l'exemple 8, 0,26 g (0,0008 mol) de carbonate de césium et de 0,113 g (0,0009 mol) de diméthylsulfate dans 5 ml d'acétone est chauffée à reflux pendant 1 heure. Après retour à température ambiante, la solution est filtrée et évaporée sous vide. Le solide est repris dans 10 ml d'éther éthylique et la solution est lavée à l'eau. Un traitement habituel de la phase organique suivi d'une purification par chromatographie sur colonne de silice en utilisant comme éluant un mélange dichlorométhane / heptane (7/3) donne le produit attendu sous forme d'huile.

| *Microanalyse élémentaire :* | | | |
|---|---|---|---|
| | *C %* | *H %* | *N %* |
| *calculé* | *72,61* | *4,93* | *4,03* |
| *trouvé* | *73,36* | *5,56* | *3,68* |

### EXEMPLE 19 : 5-Benzyl-2-hydroxy-3-(3-nitrophényl)benzaldéhyde méthyloxime

Le produit attendu est obtenu selon le procédé décrit dans l'exemple 15 en remplaçant le chlorhydrate d'hydroxylamine par le chlorhydrate de la O-méthylhydroxylamine.

| *Microanalyse élémentaire :* | | | |
|---|---|---|---|
| | *C %* | *H %* | *N %* |
| *calculé* | *69,60* | *5,01* | *7,73* |
| *trouvé* | *70,00* | *5,12* | *7,73* |

### EXEMPLE 20 : 5-Benzyl-2-méthoxy-3-(3-nitrophényl)benzaldéhyde méthyloxime

Le produit attendu est obtenu selon le procédé décrit dans l'exemple 19 à partir du composé décrit dans l'exemple 18.

| *Microanalyse élémentaire :* | | | |
|---|---|---|---|
| | *C %* | *H %* | *N %* |
| *calculé* | *70,20* | *5,36* | *7,44* |
| *trouvé* | *70,30* | *5,51* | *7,34* |

### EXEMPLE 21 : 5-Benzyl-2-hydroxy-3-(3-nitrophényl)benzonitrile

Une solution de 0,133 ml (0,00145 mol) d'oxychlorure de phosphore est additionnée goutte à goutte à une solution de 0,5 g (0,00143 mol) du composé décrit dans l'exemple 15 dans 0,3 ml de diméthylacétamide et 0,9 ml d'acétonitrile. La température interne du milieu réactionnel ne devant en aucun cas dépasser 30°C lors de l'addition. Après une demi-heure d'agitation, la solution est neutralisée avec une solution saturée de bicarbonate de sodium saturée et extraite à l'éther diéthylique. Le traitement habituel de la phase organique suivi par une chromatographie sur colonne de silice en utilisant comme éluant un mélange dichlorométhane / acétate d'éthyle (98/2) donne le produit attendu sous forme d'un solide blanc.
*Point de fusion : 148-149°C (éther de pétrole)*

| *Microanalyse élémentaire :* | | | |
|---|---|---|---|
| | *C %* | *H %* | *N %* |
| *calculé* | *72,72* | *4,27* | *8,48* |
| *trouvé* | *72,41* | *4,37* | *8,31* |

### EXEMPLE 22 : 5-Benzyl-2-méthoxy-3-(3-nitrophényl)benzonitrile

Le produit attendu est obtenu selon le procédé décrit dans l'exemple 18 à partir du composé obtenu dans l'exemple 21.

### EXEMPLE 23 : N-oxide de [5-(4-fluorobenzyl)-2-hydroxy-3-(3-nitrophényl) benzylidène]-méthylamine, chlorhydrate

A une solution chaude de 3 ml d'éthanol contenant 0,1 g (0,000284 mol) du composé décrit dans l'exemple 9 et quelques grains de tamis moléculaire, est ajouté en une seule fois 0,024 g (0,000284 mol) de chlorhydrate de N-méthylhydroxylamine. La solution est ensuite portée à reflux pendant 5 heures. Après élimination du solvant sous pression réduite, le mélange réactionnel brut est déposé sur colonne de silice en utilisant comme éluant un mélange dichlorométhane / acétate d'éthyle (9/1) et conduit au produit attendu sous forme de base que l'on transforme en chlorhydrate correspondant.
*Point de fusion : 92-95°C (ether éthylique)*

### EXEMPLE 24 : 5-(4-Fluorobenzyl)-3-(3-nitrophényl)-2-hydroxybenzoate de méthyle

### Stade A : Acide 5-(4-fluorobenzyl)-3-(3-nitrophényl)-2-hydroxybenzoïque

A une solution de 0,2 g (0,00057 mol) du composé décrit dans l'exemple 9 dans 6,5 ml de dioxane est additionnée 0,267 g (0,00222 mol) d'hydrogénophosphate de sodium et 0,082 g (0,000837 mol) d'acide sulfamique dissout dans 2,2 ml d'eau. Le mélange réactionnel est refroidi à 10°C, puis une solution de 0,067 g (0,00074 mol) de chlorite de sodium dissout dans 0,3 ml d'eau est additionnée goutte à goutte pendant 20 minutes. La solution est maintenue à 10°C pendant 1 heure puis lentement rechauffée jusqu'à température ambiante. Après 2 heures, 0,0848 g (0,00067 mol) de sulfite de sodium est additionnée en une seule fois et l'agitation est maintenue pendant 15 minutes. De l'acide chlorhydrique 1N est ajoutée jusqu'à obtention d'un pH = 2. Le dioxane est ensuite évaporé sous pression réduite et la solution aqueuse est abandonnée au réfrigérateur pendant 15 heures. Le solide, filtré et séché sous vide, conduit au produit attendu.
*Point de fusion : 210-212°C (toluène)*

### Stade B : 5-(4-Fluorobenzyl)-3-(3-nitrophényl)-2-hydroxybenzoate de méthyle

A une solution de 0,0005 mol de composé décrit au stade précédent dans un mélange de 1 ml de méthanol et 4 ml de benzène est additionnée goutte à goutte 0,00081 mol de triméthylsilyldiazométhane. Après 30 minutes d'agitation les solvants sont évaporés pour conduire au composé attendu.

| *Microanalyse élémentaire :* | | | |
|---|---|---|---|
| | *C %* | *H %* | *N %* |
| *calculé* | *66,14* | *4,23* | *3,67* |
| *trouvé* | *67,03* | *4,35* | *3,88* |

### EXEMPLE 25 : 2-Furanyl-[7-(3-nitrophényl)benzo[b]furan-5-yl]méthanone-oxime

Le produit attendu est obtenu selon le procédé décrit dans l'exemple 15 à partir du composé décrit dans l'exemple 4.
*Point de fusion : 223-225 °C*

| *Microanalyse élémentaire :* | | | |
|---|---|---|---|
| | *C %* | *H %* | *N %* |
| *calculé* | *65,52* | *3,47* | *8,04* |
| *trouvé* | *65,19* | *3,66* | *7,94* |

### EXEMPLE 26 : 7-(3-Acétylaminophényl)-5-(4-fluorobenzyl)benzo[b]furane

Le produit attendu est obtenu selon le procédé décrit dans l'exemple 2 en remplaçant au stade F l'acide 3-nitrophénylborique par l'acide 3-acétylaminophénylborique

### EXEMPLE 27 : 5-(4-Fluornbenzyl)-7-(3-trifluorométhylphényl)benzo[b]furane

Le produit attendu est obtenu selon le procédé décrit dans l'exemple 2 en remplaçant au stade F l'acide 3-nitrophénylborique par l'acide 3-trifluorométhylphénylborique.

### EXEMPLE 28 : 5-(4-Fluorobenzyl)-7-(3,5-ditrifluorométhylphényl)benzo[b]furane

Le produit attendu est obtenu selon le procédé décrit dans l'exemple 2 en remplaçant au stade F l'acide 3-nitrophénylborique par l'acide 3,5-di(trifluorométhyl)phénylborique.

### EXEMPLE 29 : 7-(3-Nitrophényl)-5-(2-thénoyl)benzo[b]furane

Le produit attendu est obtenu selon le procédé décrit dans l'exemple 4 en remplaçant au stade B le chlorure de 2-furoyle par le chlorure de 2-thénoyle.

### EXEMPLE 30 : 5-Benzyl-1-méthyl-7-(3-nitrophényl)indole

Le produit attendu est obtenu selon le procédé décrit dans l'exemple 18 à partir du composé décrit dans l'exemple 13.

| *Microanalyse élémentaire :* | | | |
|---|---|---|---|
| | *C %* | *H %* | *N %* |
| *calculé* | *77,17* | *5,30* | *8,18* |
| *trouvé* | *77,21* | *5,47* | *7,97* |

### EXEMPLE 31 : 3-(3,5-Ditrifluorométhylphényl)-5-(4-fluorobenzyl)-2-hydroxybenzaldéhyde

Le produit attendu est obtenu selon le procédé décrit dans l'exemple 8 à partir du composé décrit dans l'exemple 28.

### EXEMPLE 32 : 3-(3,5-Ditrifluorométhylphényl)-5-(4-fluorobenzyl)-2-hydroxybenzaldéhyde oxime

Le produit attendu est obtenu selon le procédé décrit dans l'exemple 15 à partir du composé décrit dans l'exemple 31.

| *Microanalyse élémentaire :* | | | |
|---|---|---|---|
| | *C %* | *H %* | *N %* |
| *calculé* | *57,78* | *3,09* | *3,06* |
| *trouvé* | *58,76* | *3,75* | *3,08* |

### EXEMPLE 33 : 7-(3-Nitrophényl)-5-(4-pyridylméthyl)benzo[b]furane, chlorhydrate

Le produit attendu est obtenu selon le procédé décrit dans l'exemple 1, en remplaçant au stade C le bromure de benzyle par le bromure de 4-pyridylméthyle.
Le chlorhydrate correspondant est obtenu par action d'une solution titrée de l'acide chlorhydrique dans l'éthanol.
*Point de fusion : 74-78°C*

| *Microanalyse élémentaire :* | | | | |
|---|---|---|---|---|
| | *C %* | *H %* | *Cl %* | *N %* |
| *calculé* | *65,49* | *4,12* | *9,67* | *7,64* |
| *trouvé* | *65,06* | *4,63* | *10,03* | *7,40* |

### EXEMPLE 34 : 2-Hydroxy-3-(3-nitrophényl)-5-(4-pyridylméthyl)benzaldéhyde

Le produit attendu est obtenu selon le procédé décrit dans l'exemple 8 à partir du composé décrit dans l'exemple 33.

### EXEMPLE 35 : 2-Hydroxy-3-(3-nitrophényl)-5-(4-pyridylméthyl)benzaldéhyde oxime, chlorhydrate

Le produit attendu est obtenu selon le procédé décrit dans l'exemple 15 à partir du composé décrit dans l'exemple 34. Le chlorhydrate correspondant est obtenu par action d'une solution titrée d'acide chlorhydrique dans l'éthanol.
*Point de fusion : 218-220°C*

| *Microanalyse élémentaire :* | | | | |
|---|---|---|---|---|
| | *C %* | *H %* | *N %* | *Cl %* |
| *calculé* | *58,05* | *4,16* | *10,69* | *10,82* |
| *trouvé* | *57,27* | *4,19* | *10,34* | *10,42* |

### EXEMPLE 36 : 2-Hydroxy-3-(3-nitrophényl)-5-(4-pyridylméthyl)benzonitrile

Le produit attendu est obtenu selon le procédé décrit dans l'exemple 21 à partir du composé décrit dans l'exemple 35.
*Point de fusion : 225°C*

| *Microanalyse élémentaire :* | | | |
|---|---|---|---|
| | *C %* | *H %* | *N %* |
| *calculé* | *68,88* | *3,95* | *12,68* |
| *trouvé* | *67,99* | *4,27* | *12,27* |

### EXEMPLE 37 : 5-(4-Fluorobenzyl)-2-méthoxy-3-(3-nitrophényl)benzaldéhyde oxime

Le produit attendu est obtenu selon le procédé décrit dans l'exemple 18 à partir du composé décrit dans l'exemple 16.

| *Microanalyse élémentaire :* | | | |
|---|---|---|---|
| | *C %* | *H %* | *N %* |
| *calculé* | *66,31* | *4,50* | *7,36* |
| *trouvé* | *65,55* | *4,60* | *7,15* |

### EXEMPLE 38 : 2,N-Dihydroxy-3'-nitro-5-(4-pyridylméthyl)biphényl-3-carboxamidine

A une solution de 0,0012 mol du composé décrit dans l'exemple 36 et 8 ml d'éthanol est ajoutée une solution de 0,0024 mol de chlorhydrate d'hydroxylamine dans 1,5 ml d'eau. Le milieu réactionnel est chauffé à 75°C pendant 72 heures.
Après refroidissement et concentration, le résidu est purifié par chromatographie sur gel de silice en utilisant comme éluant un mélange dichlorométhane / tétrahydrofurane, 8/2, pour conduire au composé attendu.
*Point de fusion : 185-186°C*

| *Microanalyse élémentaire :* | | | |
|---|---|---|---|
| | *C %* | *H %* | *N %* |
| *calculé* | *62,63* | *4,43* | *15,38* |
| *trouvé* | *63,74* | *5,06* | *15,73* |

### EXEMPLE 39 : N-Méthyl-5-(4-fluorobenzyl)-2-hydroxy-3-(3-nitrophényl)benzamide

A une solution de 10 mmol du composé décrit dans l'exemple 24, stade A, dans 30 ml de dichlorométhane, sont ajoutées 15 mmol de chlorure de thionyle à 0°C.
Après 1 heure d'agitation, 25 mmol de méthylamine sont ajoutées, et le milieu réactionnel est chauffé à reflux pendant 3 heures, refroidi et concentré. Le résidu est repris dans un mélange eau / dichlorométhane et la phase organique est séchée et concentrée pour conduire au composé attendu.

| *Microanalyse élémentaire :* | | | |
|---|---|---|---|
| | *C %* | *H %* | *N %* |
| *calculé* | *66,31* | *4,50* | *7,36* |
| *trouvé* | *66,97* | *4,93* | *7,19* |

### EXEMPLE 40 : Acide 5-(4-fluorobenzyl)-2-hydroxy-3-(3-nitrophényl) benzohydroxamique

Le produit attendu est obtenu selon le procédé décrit dans l'exemple 39 en remplaçant la méthylamine par l'hydroxylamine.

| *Microanalyse élémentaire :* | | | |
|---|---|---|---|
| | *C %* | *H %* | *N %* |
| *calculé* | *62,83* | *3,95* | *7,33* |
| *trouvé* | *60,62* | *3,89* | *6,90* |

### EXEMPLE 41 : N-(2-Chloroéthyl)-5-(4-fluorobenzyl)-3-(3-nitrophényl)-2-hydroxy. benzamide

Le produit attendu est obtenu selon le procédé décrit dans l'exemple 39 en remplaçant la méthylamine par l'éthanolamine, en présence de chlorure de thionyle.

| *Microanalyse élémentaire :* | | | | |
|---|---|---|---|---|
| | *C %* | *H %* | *N %* | *Hal %* |
| *calculé* | *61,62* | *4,23* | *6,53* | *8,27* |
| *trouvé* | *62,04* | *4,62* | *6,25* | *8,85* |

### EXEMPLE 42 : 5-[(2-Chloro-4-pyridyl)carbonyl]-7-(3-nitrophényl)benzo[b]furane

Le produit attendu est obtenu selon le procédé décrit dans l'exemple 4 en remplaçant au stade B le chlorure de 2-fuoryle par le chlorure de 2-chloroisonicotinoyle.

| *Microanalyse élémentaire :* | | | | |
|---|---|---|---|---|
| | *C %* | *H %* | *N %* | *Cl %* |
| *calculé* | *63,42* | *2,93* | *7,40* | *9,36* |
| *trouvé* | *63,53* | *2,96* | *7,42* | *9,39* |

### EXEMPLE 43 : 5-[(2-Chloro-4-pyridyl)hydroxyméthyl]-7-(3-nitrophényl)benzo[b]furane

A une solution de 0,00061 mol de composé décrit dans l'exemple 42 dans 6 ml de tétrahydrofurane est additionnée 0,0061 mol de borohydrure de sodium. Après 15 minutes, 3 ml d'une solution d'acide chlorhydrique IN sont ajoutés goutte à goutte. Le mélange est ensuite dilué dans l'éther éthylique, extrait, et la phase organique est séchée et concentrée. Le résidu obtenu est purifié par chromatographie sur gel de silice en utilisant comme éluant un mélange dichlorométhane / acétate d'éthyle, 95/5, pour conduire au composé attendu.
*Point de fusion : 178-180°C*

| *Microanalyse élémentaire :* | | | | |
|---|---|---|---|---|
| | *C %* | *H %* | *N %* | *Cl %* |
| *calculé* | *63,09* | *3,44* | *7,36* | *9,31* |
| *trouvé* | *63,28* | *3,80* | *7,05* | *8,62* |

### EXEMPLE 44 : 7-(3-Nitrophényl)-5-[(2-thiényl)hydroxyméthyl]benzo[b]furane

Le produit attendu est obtenu selon le procédé décrit dans l'exemple 43 à partir du composé décrit dans l'exemple 29.

| *Microanalyse élémentaire :* | | | | |
|---|---|---|---|---|
| | *C %* | *H %* | *N %* | *S %* |
| *calculé* | *64,95* | *3,73* | *3,99* | *9,13* |
| *trouvé* | *65,60* | *3,88* | *3,94* | *8,84* |

### EXEMPLE 45 : 7-(3-Nitrophényl)-5-(2-thiénylméthyl)benzo[b]furane

Le produit attendu est obtenu selon le procédé décrit dans l'exemple 1 en remplaçant au stade C le bromure de benzyle par le 2-chlorométhylthiophène.

| *Microanalyse élémentaire :* | | | | |
|---|---|---|---|---|
| | *C %* | *H %* | *N %* | *S %* |
| *calculé* | *68,04* | *3,91* | *4,18* | *9,56* |
| *trouvé* | *68,47* | *4,20* | *4,26* | *9,26* |

### EXEMPLE 46 : 7-(3-Nitrophényl)-5-(4-pyridylvinyl)benzo[b]furane, chlorhydrate

Le produit attendu est obtenu selon le procédé décrit dans l'exemple 7 en remplaçant au stade A, le tetrakistriphénylphosphine palladium par l'acétate de palladium (II), et l'allytributylétain par la 4-vinylpyridine. Le chlorhydrate correspondant est obtenu par action d'une solution titrée d'acide chlorhydrique dans l'éthanol.
*Point de fusion : > 250°C*

| *Microanalyse élémentaire :* | | | | |
|---|---|---|---|---|
| | *C %* | *H %* | *Cl %* | *N %* |
| *calculé* | *66,58* | *3,99* | *9,36* | *7,39* |
| *trouvé* | *66,16* | *4,24* | *8,97* | *7,27* |

### EXEMPLE 47 : 2-Iodo-6-(3-nitrophényl)-4-(4-pyridylméthyl)phénol

### Stade A : 2-Bromo-4-(4-pyridylméthyl)aniline

Une solution de 18,34 g (0,103 mol) de N-bromosuccinimide dans 60 ml de diméthylformamide est additionnée goutte à goutte, à l'abri de la lumière, à une solution de 20 g (0,1084 mol) de 4-(4-pyridylméthyl)aniline dans 180 ml de diméthylformamide. Après 1 heure, le solvant est évaporé sous pression réduite et le solide est repris par 200 ml de dichlorométhane. La solution est alors lavée par 100 ml d'eau puis 100 ml d'une solution de chlorure de sodium saturée. Après le traitement habituel de la phase organique, l'huile est reprise par 500 ml d'éther diéthylique et le tout est abandonné au réfrigéateur pendant 2 jours. Après filtration, le produit attendu est obtenu.
*Point de fusion : 102-104°C*

### Stade B : 2-(3-Nitrophényl)-4-(4-pyridylméthyl)aniline

Une solution de 25 g (0,095 mol) de composé décrit au stade précédent, 23,8 g (0,1426 mol) d'acide 3-nitrophénylborique, 26,3 g (0,19 mol) de carbonate de potassium et de 3,9 g (0,00475 mol) de complexe de palladium dans 320 ml de diméthylformamide est chauffée à 80°C sous atmosphère inerte pendant 16 heures. Après retour à température ambiante, la solution est diluée par 200 ml de diméthylformamide puis est filtrée sur célite. Le filtrat est évaporé sous pression réduite et est ensuite repris par 300 ml d'acétate d'éthyle. Un traitement habituel de la phase organique suivi d'une purification par chromatographie sur colonne de silice (éluant : dichlorométhane - méthanol 97/3) permet d'obtenir le produit du titre.
*Point de fusion : 133-135°C*

### Stade C : 2-(3-Nitrophényl)-4-(4-pyridylméthyl)phénol

Une solution de 13,7 g (0,045 mol) de composé décrit au stade précédent et de 47 ml d'acide sulfurique à 35% est refroidie à 0°C. Le sulfate de pyridinium est dilué à l'aide de 50 ml d'eau et maintenu à 0°C. On additionne alors goutte à goutte et sous la surface, une solution de 4,06 g (0,058 mol) de nitrite de sodium et de 50 ml d'eau en maintenant la température inférieure à +5°C dans le réacteur. Après 15 minutes d'agitation à 0°C, la solution de sel de diazonium est ajouttée goutte à goutte à une solution bouillante de 25 ml d'acide sulfurique à 35% (la température du milieu réactionnel devant toujours être supérieure à 70°C). Lorsque l'addition est terminée, la solution bouillante est versée sur 80 ml d'une solution d'ammoniaque concentrée (28%) glacée. La phase aqueuse basique est extraite au dichlorométhane contenant 5 % de méthanol. Un traitement habituel de la phase organique suivi d'une purification par chromatographie sur colonne de silice (éluant : dichlorométhane - méthanol : 95/5) permet d'obtenir le produit attendu.
*Point de fusion : 122-124°C*

### Stade D : 2-Iodo-6-(3-nitrophényl)-4-(4-pyridylméthyl)phénol

A une solution de 0,026 mol de composé décrit au stade précédent dans 675 ml d'un mélange 2/l dichlorométhane - méthanol est additionnée, en une seule fois, 0,0654 mol de bicarbonate de sodium puis 0,03 mol benzyltriméthylammonium dichloroiodate. Après 2 heures le mélange est filtré puis évaporé. Le solide est repris par 100 ml de dichlorométhane et lavé par 2 fois 50 ml d'une solution de bicarbonate de sodium à 5%. Le traitement habituel de la phase organique suivi d'une purification par chromatographie sur gel de silice, permet d'obtenir le produit du titre.
*Point de fusion : 168-170°C*

| *Microanalyse élémentaire :* | | | | |
|---|---|---|---|---|
| | *C %* | *H %* | *N %* | *I %* |
| *calculé* | *50,02* | *3,03* | *6,48* | *29,36* |
| *trouvé* | *49,64* | *3,32* | *6,22* | *28,94* |

### EXEMPLE 48 : 2-Hydroxy-3-(3-nitrophényl)-5-(4-pyridylméthyl)acétophénone, chlorhydrate

Une solution de 0,0046 mol de composé décrit dans l'exemple 47, 0,009 mol d'éthoxyvinyl tributylétain, 0,028 mol de chlorure de lithium et 0,0005 mol de tétrakistriphénylphosphine palladium (0) dans 40 ml de tétrahydrofurane est chauffé à 65°C pendant 76 heures. Après refroidissement, le milieu est dilué avec de l'acétate d'éthyle. La phase organique est séchée, concentrée et purifiée en utilisant comme éluant un mélange dichlorométhane / acétate d'éthyle, 7/3 pour conduire au composé attendu. Le chlorhydrate correspondant est obtenu par action d'une solution titrée d'acide chlorhydrique dans l'éthanol.
*Point de fusion : 219-221°C*

| *Microanalyse élémentaire :* | | | | |
|---|---|---|---|---|
| | *C %* | *H %* | *N %* | *Cl %* |
| *calculé* | *62,42* | *4,45* | *7,28* | *9,21* |
| *trouvé* | *61,93* | *4,30* | *7,05* | *9,19* |

### EXEMPLE 49 : 2-Hydroxy-3-(3-nitrophényl)-5-(4-pyridylméthyl)acétophénone oxime

Le produit attendu est obtenu selon le procédé décrit dans l'exemple 15 à partir du composé décrit dans l'exemple 48.
*Point de fusion : 209-211°C*

| *Microanalyse élémentaire :* | | | |
|---|---|---|---|
| | *C %* | *H %* | *N %* |
| *calculé* | *66,11* | *4,72* | *11,56* |
| *trouvé* | *66,15* | *5,03* | *10,89* |

### EXEMPLE 50 : 7-(3-Chlorophényl)-5-(4-pyridylméthyl)benzo[b]furane

Le produit attendu est obtenu selon le procédé décrit dans l'exemple 33 en remplaçant l'acide 3-nitrophénylborique par l'acide 3-chlorophénylborique.

### EXEMPLE 51 : 3-(3-Chlorophényl)-2-hydroxy-5-(4-pyridylméthyl)benzaldéhyde

Le produit attendu est obtenu selon le procédé décrit dans l'exemple 8 à partir du composé décrit dans l'exemple 50.

### EXEMPLE 52 : 3-(3-Chlorophényl)-2-hydroxy-5-(4-pyridylméthyl)benzaldéhyde oxime

Le produit attendu est obtenu selon le procédé décrit dans l'exemple 15 à partir du composé décrit dans l'exemple 51.
*Point de fusion : 124-125°C*

| *Microanalyse élémentaire :* | | | | |
|---|---|---|---|---|
| | *C %* | *H %* | *Cl %* | *N %* |
| *calculé* | *60,81* | *4,30* | *9,45* | *7,47* |
| *trouvé* | *61,39* | *4,60* | *9,67* | *7,36* |

### EXEMPLE 53 : 5-Benzyl-2-hydroxy-3-(3-nitrophényl)benzaldéhyde hydrazone

Une solution de 0,006 mol du composé décrit dans l'exemple 8 et de 0,012 mol d'hydrazine dans 20 ml de toluène est chauffé à reflux pendant 30 minutes. Après refroidissement, le produit attendu qui précipite est filtré puis séché.

| *Microanalyse élémentaire :* | | | |
|---|---|---|---|
| | *C %* | *H %* | *N %* |
| *calculé* | *69,15* | *4,93* | *12,10* |
| *trouvé* | *69,45* | *5,16* | *11,64* |

### EXEMPLE 54 : 7-(3-Nitrophényl)-5-(4-pyridylthlo)benzo[b]furane

### Stade A : 7-Méthoxy-5-(4-pyridylthio)benzo[b]furane

Une solution de 0,044 mol de composé décrit dans l'exemple 1, stade B, 0,046 mol de 4-mercaptopyridine, 0,088 mol de tert-butylate de sodium et de 0,0017 mol de tétrakistriphénylphosphine palladium (0) dans 180 ml de n-butanol est chauffée à 105°C pendant 6 heures. Après refroidissement, le mélange réactionnel est concentré, et le résidu est purifié par chromatographie sur gel de silice en utilisant comme éluant un mélange dichlorométhane / acétate d'éthyle, 8/2, pour conduire au composé attendu.
*Point de fusion : 81-83°C*

### Stade B : 7-Hydroxy-5-(4-pyridylthio)benzo[b]furane

Le produit attendu est obtenu selon le procédé décrit dans l'exemple 1, stade D, à partir du composé décrit au stade précédent.
*Point de fusion : 154-156°C*

### Stade C : 5-(4-Pyridylthio)-7-trifluorométhanesulfonyloxybenzo[b]furane

Le produit attendu est obtenu selon le procédé décrit dans l'exemple 1, stade E, à partir du composé décrit au stade précédent.

### Stade D : 7-(3-Nitrophényl)-5-(4-pyridylthio)benzo[b]furane

Le produit attendu est obtenu selon le procédé décrit dans l'exemple 1, stade F, à partir du composé décrit au stade précédent.
*Point de fusion : 135-137°C*

| *Microanalyse élémentaire :* | | | | |
|---|---|---|---|---|
| | *C %* | *H %* | *N %* | *Cl %* |
| *calculé* | *65,51* | *3,47* | *8,04* | *9,20* |
| *trouvé* | *65,68* | *3,63* | *7,90* | *8,75* |

### EXEMPLE 55 : 4-Benzyl-2-(but-1-enyl)-6-(3-nitrophényl)phénol

A une solution 0,014 mol de bromure de n-propyltriphénylphosphonium dans 25 ml de tetrahydrofurane est additionnée goutte à goutte, à 4°C, une solution de 9 ml de n-butyllithium 1,6 M dans l'hexane. Après 30 minutes à 4°C, puis 30 minutes à 20°C, une solution de 0,0048 mol de composé décrit dans l'exemple 8, dans 5 ml de tétrahydrofurane est additionnée goutte à goutte. Le mélange réactionnel est ensuite acidifié par 15 ml d'une solution d'acide chlorhydrique 1N, dilué avec l'éther et extrait. La phase organique est concentrée et purifiée par chromatographie sur gel de silice en utilisant comme éluant un mélange dichlorométhane / heptane 6/4, pour conduire au composé attendu.

| *Microanalyse élémentaire :* | | | |
|---|---|---|---|
| | *C %* | *H %* | *N %* |
| *calculé* | *76,86* | *5,89* | *3,90* |
| *trouvé* | *76,53* | *6,06* | *3,91* |

En utilisant le procédé décrit dans l'exemple 55 et en remplaçant le bromure de n-propyltriphénylphosphonium par l'halogénure de triphénylphosphonium correspondant, on obtient les composés des exemples 56 et 57.

### EXEMPLE 56 : 4-Benzyl-2-(2-méthoxyvinyl)-6-(3-nitrophényl)phénol

| *Microanalyse élémentaire :* | | | |
|---|---|---|---|
| | *C %* | *H %* | *N %* |
| *calculé* | *73,12* | *5,30* | *3,88* |
| *trouvé* | *73,31* | *5,14* | *4,40* |

### EXEMPLE 57 : 4-Benzyl-2-(2-méthylthiovinyl)-6-(3-nitrophényl)phénol

| *Microanalyse élémentaire :* | | | | |
|---|---|---|---|---|
| | *C %* | *H %* | *N %* | *S %* |
| *calculé* | *70,01* | *5,07* | *3,71* | *8,49* |
| *trouvé* | *69,86* | *5,14* | *3,70* | *8,13* |

En utilisant les procédés décrits précédemment, les composés suivants sont obtenus :

### EXEMPLE 58 : 2-(2-Méthoxyvinyl)-6-(3-nitrophényl)-5-(4-pyridylméthyl)phénol

### EXEMPLE 59 : 7-(3-Nitrophényl)-5-(4-pyridylméthyl)benzo[b]thiophène

### EXEMPLE 60 : 2-Mercapto-3-(3-nitrophényl)-5-(4-pyridylméthyl)benzaldéhyde oxime

### EXEMPLE 61 : 2-Méthylthio-3-(3-nitrophényl)-5-(4-pyridylméthyl)benzaldéhyde oxime

### EXEMPLE 62 : 5-(1H-2-Imidazolylméthyl)-7-(3-nitrophényl)benzo[b]furane

### EXEMPLE 63 : 2-Hydroxy-5-(1H-2-imidazolylméthyl)-3-(3-nitrophényl) benzaldéhyde oxime

### EXEMPLE 64 : 5-[(1-Méthyl-2-imidazolyl)méthyl]-7-(3-nitrophényl)benzo[b]furane

### EXEMPLE 65 : 2-Hydroxy-5-[(1-méthyl-2-imidazolyl)méthyl]-3-(3-nitrophényl)benzaldéhyde oxime

### EXEMPLE 66 : 5-[(1-Méthyl-5-imidazolyl)méthyl]-7-(3-nitrophényl)benzo[b]furane

### EXEMPLE 67 : 2-Hydroxy-5-[(1-méthyl-5-imidazolyl)méthyl]-3-(3-nitrophényl) benzaldéhyde oxime

### EXEMPLE 68 : 5-[(1-Méthyl-4-imidazolyl)méthyl]-7-(3-nitrophényl)benzo[b]furane

### EXEMPLE 69 : 2-Hydroxy-5-[(1-méthyl-4-imidazolyl)méthyl]-3-(3-nitrophényl) benzaldéhyde oxime

### EXEMPLE 70 : 7-(3-Nitrophényl)-5-(1H-3-pyrazolylméthyl)benzo[b]furanes EXEMPLE 71 : 2-Hydroxy-3-(3-nitrophényl)-5-(1H-3-pyrazolylméthyl)benzaldéhyde oxime

### EXEMPLE 72 : 5-[(2-Méthyl-3-pyrazolyl)méthyl]-7-(3-nitrophényl)benzo[b]furane

### EXEMPLE 73 : 2-Hydroxy-5-[(2-méthyl-3-pyrazolyl)méthyl]-3-(3-nitrophényl) benzaldéhyde oxime

### EXEMPLE 74 : 7-(3-Nitrophényl)-5-(2-quinolylméthyl)benzo[b]furane

### EXEMPLE 75 : 2-Hydroxy-3-(3-nitrophényl)-5-(2-quinolylméthyl)benzaldéhyde oxime

### EXEMPLE 76 : 7-(3-Nitrophényl)-5-(3-quinolylméthyl)benzo[b]furane

### EXEMPLE 77 : 2-Hydroxy-3-(3-nitrophényl)-5-(3-quinolylméthyl)benzaldéhyde oxime

### EXEMPLE 78 : 7-(3-Nitrophényl)-5-(2-pyrazinylméthyl)benzo[b]furane

### EXEMPLE 79 : 2-Hydroxy-3-(3-nitrophényl)-5-(2-pyrazinylméthyl)benzaldéhyde oxime

### EXEMPLE 80 : 7-(4-Pyridyl)-5-(4-pyridylméthyl)benzo[b]furane

### EXEMPLE 81 : 2-Hydroxy-3-(4-pyridyl)-5-(4-pyridylméthyl)benzaldéhyde oxime

### EXEMPLE 82 : 7-(3-Pyridyl)-5-(4-pyridylméthyl)benzo[b]furane

### EXEMPLE 83 : 2-Hydroxy-3-(3-pyridyl)-5-(4-pyridylméthyl)benzaldéhyde oxime

### EXEMPLE 84 : 7-(2-Pyridyl)-5-(4-pyridylméthyl)benzo[b]furane

### EXEMPLE 85 : 2-Hydroxy-3-(2-pyridyl)-5-(4-pyridylméthyl)benzaldéhyde oxime

### EXEMPLE 86 : 7-(3-Nitrophényl)-5-(3-pyridylméthyl)benzo[b]furane

### EXEMPLE 87 : 2-Hydroxy-3-(3-nitrophényl)-5-(3-pyridylméthyl)benzaldéhyde oxime

### EXEMPLE 88 : 7-(3-Nitrophényl)-5-(2-pyridylméthyl)benzo[b]furane

### EXEMPLE 89 : 2-Hydroxy-3-(3-nitrophényl)-5-(2-pyridylméthyl)benzaldéhyde oxime

### EXEMPLE 90 : 7-(3-Nitrophényl)-5-[2-(4-pyridyl)éthyl]benzo[b]furane

### EXEMPLE 91 : 2-Hydroxy-3-(3-nitrophényl)-5-[2-(4-pyridyl)éthyl]benzaldéhyde oxime

### EXEMPLE 92 : 7-(3-Nitrophényl)-5-phénoxybenzo[b]furane

### EXEMPLE 93 : 2-Hydroxy-3-(3-nitrophényl)-5-phénoxybenzaldéhyde oxime

### EXEMPLE 94 : 7-(3-Nitrophényl)-5-(4-pyridyloxy)benzo[b]furane

### EXEMPLE 95 : 2-Hydroxy-3-(3-nitrophényl)-5-(4.pyridyloxy)benzaldéhyde oxime

### EXEMPLE 96 : 2-Acétylamino-4-benzyl-6-(3-nitrophényl)phénol

### EXEMPLE 97 : 4-Benzyl-2-formamido-6-(3-nitrophényl)phénol

### EXEMPLE 98 : 2-Acétylamino-4-(4-pyridylméthyl)-6-(3-nitrophényl)phénol

### EXEMPLE 99 : 2-Formamido-4-(4-pyridylméthyl)-6-(3-nitrophényl)phénol

### EXEMPLE 100 : N-Méthyl-5-benzyl-2-hydroxy-3-(3-nitrophényl)benzaldéhyde hydrazone

### Point de fusion : 154-156°C

### Etude pharmacologique des dérivés de l'invention

### EXEMPLE 101 : Mesure de l'activité PDE

Les cellules U937 sont cultivées dans un milieu de culture (RPMI) contenant 10 % de sérum de veau foetal. Brièvement, les cellules sont lysées puis ensuite centrifugées (100 000 g, 60 min, 4°C) et le surnageant est récupéré en vue d'une séparation par HPLC des différentes formes de PDE (C. LUGNIER et V.B. SCHINI, Biochem. Pharmacol. Vol. 39, p. 75-84, 1990). L'activité PDE est mesurée par l'apparition de [³H]5' AMP résultant de l'hydrolyse du [³H]AMP cyclique. La PDE et le [³H]AMP cyclique (1µCi/mL) sont incubés 30 minutes à 30°C. La radioactivité est mesurée au moyen d'un compteur à scintillation liquide (Beckman LS 1701).

La PDE IV est caractérisée par :
- l'hydrolyse de l'AMP cyclique,
- l'absence d'inhibition par le GMP cyclique de l'hydrolyse d'AMP cyclique,
- l'inhibition par le rolipram, molécule de référence.

Les composés sont étudiés à deux concentrations (10⁻⁷M et 10⁻⁵M) en duplicate. Les résultats sont exprimés en % d'inhibition de l'activité phosphodiestérasique. Les meilleurs composés de la dite invention présentent une inhibition à 10⁻⁷M égale ou supérieure à 80 %.

### EXEMPLE 102 : Composition pharmaceutique

| Formule de préparation pour 1000 comprimés dosés à 100 mg | |
|---|---|
| Composé de l'exemple 15 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) : 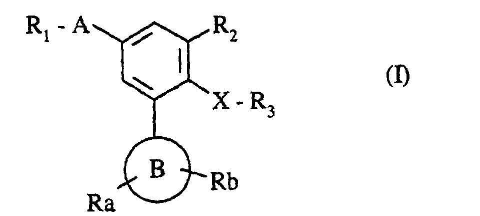 dans laquelle :
R₁ représente un groupement cycloalkyle (C₃-C₇) substitué ou non, un groupement phényle substitué ou non, un groupement naphtyle substitué ou non, un groupement hétérocyclique, saturé ou insaturé, substitué ou non, mono ou bicyclique contenant de 1 à 3 hétéroatomes choisis parmi azote, oxygène ou soufre, un groupement alkyle (C₁-C₆) linéaire ou ramifié substitué ou non, ou un groupement alkényle (C₁-C₆) linéaire ou ramifié substitué ou non;
A représente une liaison (à la condition que dans ce cas R₁ soit différent d'un groupement alkyle C₁ ou C₂), un atome d'oxygène, de soufre, un groupement 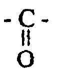 un groupement 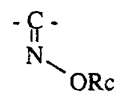 ou un groupement 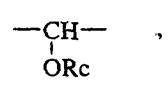 (dans lesquels Rc représente un atome d'hydrogène, un groupement alkyle (C₁-C₆) linéaire ou ramifié ou un groupement cycloalkyle (C₃-C₇)),
R₂ représente un atome d'halogène, un groupement alkyle (C₁-C₆) linéaire ou ramifié substitué ou non, alkényle (C₁-C₆) linéaire ou ramifié substitué ou non, alkynyle (C₁-C₆) linéaire ou ramifié substitué ou non, cycloalkyle (C₃-C₇) substitué ou non, formyle, carboxy, alkylcarbonyle (C₁-C₆) linéaire ou ramifié, cycloalkylcarbonyle (C₃-C₇), alkoxycarbonyle (C₁-C₆) linéaire ou ramifié, cycloalkyloxycarbonyle (C₃-C₇), carbamoyle (éventuellement substitué par un ou deux groupements alkyle (C₁-C₆) linéaire ou ramifié, cycloalkyle (C₃-C₇), hydroxy, alkoxy (C₁-C₆) linéaire ou ramifié, cycloalkyloxy (C₃-C₇) ou formant ensemble avec l'atome d'azote qui les porte un cycle de 5 à 7 chaînons contenant de 1 à 3 hétéroatomes choisis parmi azote, oxygène et soufre), amino (éventuellement substitué par un ou deux groupements alkyle (C₁-C₆) linéaire ou ramifié, acyle (C₁-C₆) linéaire ou ramifié, cycloalkyle (C₃-C₇) ou formant ensemble avec l'atome d'azote qui les porte un cycle de 5 à 7 chaînons contenant de 1 à 3 hétéroatomes choisis parmi azote, oxygène et soufre), formamido, cyano, amidino (éventuellement substitué par un ou deux groupements alkyle (C₁-C₆) linéaire ou ramifié, cycloalkyle (C₃-C₇), ou formant ensemble avec l'atome d'azote qui les porte un cycle de 5 à 7 chaînons contenant de 1 à 3 hétéroatomes choisis parmi azote, oxygène et soufre), hydroxyaminométhyle (éventuellement substitué, indépendamment sur l'atome d'azote ou d'oxygène, par un groupement alkyle (C₁-C₆) linéaire ou ramifié, cycloalkyle (C₃-C₇), acyle (C₁-C₆) linéaire ou ramifié), amide-oxime (éventuellement substitué, indépendamment sur l'atome d'azote ou d'oxygène par un groupement alkyle (C₁-C₆) linéaire ou ramifié, cycloalkyle (C₃-C₇), ou formant avec l'atome d'azote qui les porte un cycle de 5 à 7 chaînons contenant de 1 à 3 hétéroatomes choisis parmi azote, oxygène et soufre), hydrazono (éventuellement substitué par un ou deux groupements alkyles (C₁-C₆) linéaires ou ramifiés, cycloalkyles (C₃-C₇), ou formant avec l'atome d'azote qui les porte un cycle de 5 à 7 chaînons contenant de 1 à 3 hétéroatomes choisis parmi azote, oxygène et soufre), ou un groupement choisi parmi : 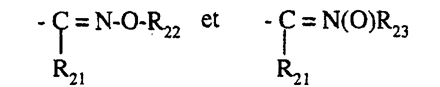 dans lesquels R₂₁ représente un atome d'hydrogène, un groupeemnt alkyle (C₁-C₆) linéaire ou ramifié, ou un groupement cycloalkyle (C₃-C₇),
R₂₂ représente un atome d'hydrogène ou un groupement alkyle (C₁-C₆) linéaire ou ramifié cycloalkyle (C₃-C₇), ou acyle (C₁-C₆) linéaire ou ramifié,
R₂₃ représente un groupement alkyle (C₁-C₆) linéaire ou ramifié, ou cycloalkyle (C₃-C₇),
R₃ représente un atome d'hydrogène, un groupement alkyle (C₁-C₆) linéaire ou ramifié, un groupement cycloalkyle (C₃-C₇) ou un groupement acyle (C₁-C₆) linéaire ou ramifié, ou bien 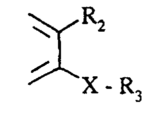 forment ensemble le cycle 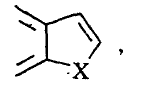
X représente un atome d'oxygène, de soufre ou un groupement NR" (dans lequel R" représente un atome d'hydrogène, un groupement alkyle (C₁-C₆) linéaire ou ramifié, cycloalkyle (C₃-C₇) ou acyle (C₁-C₆) linéaire ou ramifié),
le cycle B représente un cycle phényle ou pyridyle,
Ra, Rb, identiques ou différents, représentent un atome d'hydrogène, d'halogène, un groupement alkyle (C₁-C₆) linéaire ou ramifié, hydroxy, alkoxy (C₁-C₆) linéaire ou ramifié, carboxy, polyhalogénoalkyle (C₁-C₆) linéaire ou ramifié, cyano, nitro, alkoxycarbonyle (C₁-C₆) linéaire ou ramifié, cycloalkyloxycarbonyle (C₃-C₇), amino (éventuellement substitué par un ou deux groupements alkyle (C₁-C₆) linéaire ou ramifié, acyle (C₁-C₆) linéaire ou ramifié ou cycloalkyle (C₃-C₇)), carbamoyle (éventuellement substitué par un ou deux groupements alkyle (C₁-C₆) linéaire ou ramifié, cycloalkyle (C₃-C₇) ou alkoxy (C₁-C₆) linéaire ou ramifié), sulfo, alkylsulfonyle (C₁-C₆) linéaire ou ramifié, cycloalkylsulfonyle (C₃-C₇) ou aminosulfonyle (éventuellement substitué par un ou deux groupements alkyle (C₁-C₆) linéaire ou ramifié ou cycloalkyle (C₃-C₇),
étant entendu que
- lorsque A représente une liaison ou un atome d'oxygène, B représente un cycle phényle, R₁ représente un groupement cycloalkyle (C₃-C₇) substitué ou non, un groupement phényle substitué ou non, un groupement naphtyle substitué ou non, X représente un atome d'oxygène ou un groupement NR" (dans lequel R" représente un atome d'hydrogène ou un groupement alkyle (C₁-C₆) linéaire ou ramifié) et R₃ représente un atome d'hydrogène ou un groupement alkyle (C₁-C₆) linéaire ou ramifié, alors R₂ est différent d'un groupement carboxy,
- que par groupement alkyle substitué, aikényle substitué, alkynyle substitué ou cycloalkyle substitué, on entend substitué par un ou plusieurs atomes d'halogènes ou groupements alkoxy (C₁-C₆) linéaire ou ramifié, alkylthio (C₁-C₆) linéaire ou ramifié, cycloalkyle (C₃-C₇), phényle substitué ou non, naphtyle substitué ou non ou hétérocyclique substitué ou non (comme par exemple un groupement pyridyle). Un groupement alkyle saturé ou insaturé substitué préféré est le groupement benzyle substitué ou non,
- que par groupement phényle substitué, naphtyle substitué ou hétérocyclique substitué ou benzyle substitué, on entend substitué par un ou plusieurs atomes d'halogène ou groupements alkyle (C₁-C₆) linéaire ou ramifié, alkoxy (C₁-C₆) linéaire ou ramifié, ou trihalogénométhyle.
leurs énantiomères, diastéroisomères ainsi que leurs sels d'addition à un acide ou à une base pharmaceutiquement acceptable.

2. Composés de formule (I) selon la revendication 1 tels que le cycle B représente un cycle phényle.

3. Composés de formule (I) selon la revendication 1 tels que le le cycle B représente un cycle pyridyle.

4. Composés de formule (I) selon la revendication 1 tels que X représente un atome d'oxygène.

5. Composés de formule (I) selon la revendication 1 tels que R₁ représente un groupement alkyle substitué par un groupement phényle substitué ou non.

6. Composés de formule (I) selon la revendication 1 tels que R₁ représente un groupement alkyle substitué par un groupement hétérocyclique, saturé ou insaturé, substitué ou non, contenant de 1 à 3 hétéroatomes choisis parmi oxygène; azote ou soufre.

7. Composés de formule (I) selon la revendication 6 tels que R₁ représente un groupement alkyle substitué par un groupement pyridyle substitué ou non.

8. Composés de formule (I) selon la revendication 6 tels que R₁ représente un groupement hétérocyclique, saturé ou insaturé, substitué ou non, contenant de 1 à 3 hétéroatomes choisis parmi oxygène, azote ou soufre.

9. Composés de formule (I) selon la revendication 1 tels que R₂ représente un atome d'halogène, un groupement alkyle (C₁-C₆) linéaire ou ramifié substitué ou non, alkényle (C₁-C₆) linéaire ou ramifié substitué ou non, alkynyle (C₁-C₆) linéaire ou ramifié substitué ou non, cycloalkyle (C₃-C₇) substitué ou non, formyle, carboxy, alkylcarbonyle (C₁-C₆) linéaire ou ramifié, cycloalkylcarbonyle (C₃-C₇), alkoxycarbonyle (C₁-C₆) linéaire ou ramifié, cycloalkyloxycarbonyle (C₃-C₇), carbamoyle (éventuellement substitué par un ou deux groupements alkyle (C₁-C₆) linéaire ou ramifié, cycloalkyle (C₃-C₇), hydroxy, alkoxy (C₁-C₆) linéaire ou ramifié, cycloalkyloxy (C₃-C₇) ou formant ensemble avec l'atome d'azote qui les porte un cycle de 5 à 7 chaînons contenant de 1 à 3 hétéroatomes choisis parmi azote, oxygène et soufre), amino (éventuellement substitué par un ou deux groupements alkyle (C₁-C₆) linéaire ou ramifié, acyle (C₁-C₆) linéaire ou ramifié, cycloalkyle (C₃-C₇) ou formant ensemble avec l'atome d'azote qui les porte un cycle de 5 à 7 chaînons contenant de 1 à 3 hétéroatomes choisis parmi azote, oxygène et soufre), formamido, cyano, amidino (éventuellement substitué par un ou deux groupements alkyle (C₁-C₆) linéaire ou ramifié, cycloalkyle (C₃-C₇), ou formant ensemble avec l'atome d'azote qui les porte un cycle de 5 à 7 chaînons contenant de 1 à 3 hétéroatomes choisis parmi azote, oxygène et soufre), hydroxyaminométhyle (éventuellement substitué, indépendamment sur l'atome d'azote ou d'oxygène, par un groupement alkyle (C₁-C₆) linéaire ou ramifié, cycloalkyle (C₃-C₇) ou acyle (C₁-C₆) linéaire ou ramifié), amide-oxime (éventuellement substitué, indépendamment sur l'atome d'azote ou d'oxygène par un groupement alkyle (C₁-C₆) linéaire ou ramifié, cycloalkyle (C₃-C₇), ou formant avec l'atome d'azote qui les porte un cycle de 5 à 7 chaînons contenant de 1 à 3 hétéroatomes choisis parmi azote, oxygène et soufre), hydrazono (éventuellement substitué par un ou deux groupements alkyles (C₁-C₆) linéaires ou ramifiés, cycloalkyles (C₃-C₇), ou formant avec l'atome d'azote qui les porte un cycle de 5 à 7 chaînons contenant de 1 à 3 hétéroatomes choisis parmi azote, oxygène et soufre), ou un groupement choisi parmi : 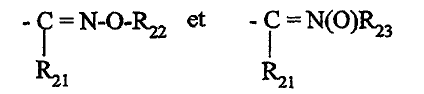 dans lesquels R₂₁ représente un atome d'hydrogène, un groupement alkyle (C₁-C₆) linéaire ou ramifié, ou un groupement cycloalkyle (C₃-C₇),
R₂₂ représente un atome d'hydrogène ou un groupement alkyle (C₁-C₆) linéaire ou ramifié, cycloalkyle (C₃-C₇), ou acyle (C₁-C₆) linéaire ou ramifié,
R₂₃ représente un groupement alkyle (C₁-C₆) linéaire ou ramifié, ou cycloalkyle (C₃-C₇).

10. Composés de formule (I) selon la revendication 1 tels que 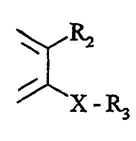 forment ensemble le cycle

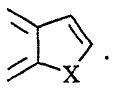

11. Composés de formule (I) selon la revendication 10 tels que X représente un atome d'oxygène.

12. Composé de formule (I) selon la revendication 1 qui est le 2-hydroxy-3-(3-nitrophényl)-5-pyridylbenzaldéhyde oxime,
ses isomères ainsi que ses sels d'addition à un acide ou à une base pharmaceutiquement acceptable.

13. Composé de formule (I) selon la revendication 1 qui est le 5-benzyl-2-hydroxy-3-(3-nitrophényl)benzaldéhyde oxime,
ses isomères ainsi que ses sels d'addition à une base pharmaceutiquement acceptable.

14. Composé de formule (I) selon la revendication 1 qui est le 7-(3-nitrophényl)-5-(4-pyridylméthyl)benzo[b]furane,
ses isomères ainsi que ses sels d'addition à un acide pharmaceutiquement acceptable.

15. Procédé de préparation des composés de formule (I) selon la revendication 1 caractérisé en ce que l'on utilise comme produit de départ un composé de formule (II): 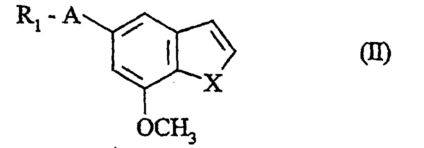 dans laquelle R₁, A et X ont la même signification que dans la formule (I),
que l'on soumet à l'action du tribromure de bore en milieu dichlorométhane, pour conduire au composé de formule (III) : 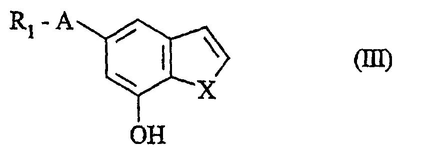 dans laquelle R₁, A et X ont la même signification que dans la formule (I),
que l'on soumet à l'action de l'anhydride trifluorométhanesulfonique dans de la pyridine, pour conduire au composé de formule (IV) : 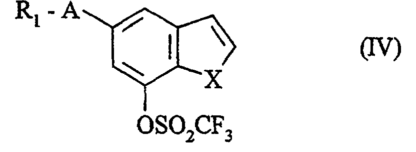 dans laquelle R₁, A et X ont la même signification que dans la formule (I),
qui subit l'action d'un acide borique de formule (V): 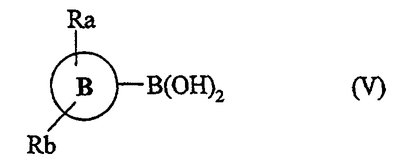 dans laquelle B, Ra et Rb sont tels que définis dans la formule (I),
pour conduire au composé de formule (I/a), cas particulier des composés de formule (I): 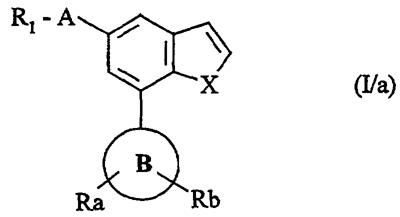 dans laquelle R₁, A, Ra, Rb, B et X ont la même signification que dans la formule (I),
composé de formule (I/a), qui subit, si on le désire, une coupure oxydante, pour conduire au composé de formule (I/b), cas particulier des composés de formule (I) : 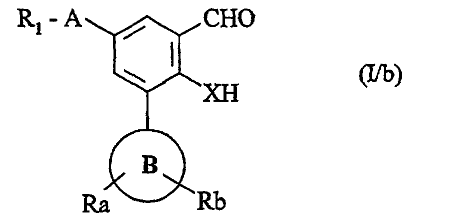 dans laquelle R₁, A, Ra, Rb, B et X ont la même signification que dans la formule (I),
composé de formule (I/b) qui peut subir :
**a** soit l'action du composé de formule (VI) en milieu basique :
R₂₂O - NH₂ (VI)
dans laquelle R₂₂ représente un atome d'hydrogène ou un groupement alkyle (C₁-C₆) linéaire ou ramifié, cycloalkyle (C₃-C₇) ou acyle (C₁-C₆) linéaire ou ramifié, pour conduire au composé de formule(I/c), cas particulier des composés de formule (I) : 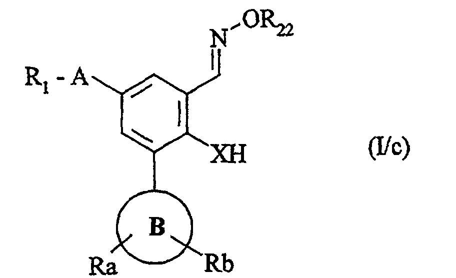 dans laquelle R₁, A, Ra, Rb, B, X et R₂₂ sont tels que définis précédemment,
composé de formule (I/c), que l'on transforme, éventuellement, lorsque R₂₂ est différent d'un groupement acyle, en milieu acide, en composé de formule (I/d), cas particulier des composés de formule (I): 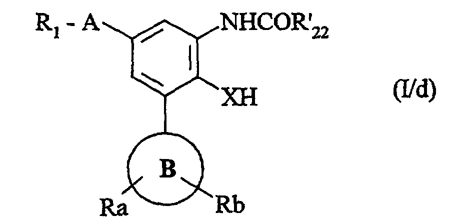 dans laquelle R₁, A, Ra, Rb, B et X ont la même signification que précédemment, et R'₂₂ différent d'un groupement acyle à la même définition que R₂₂,
**b** soit l'action, en milieu alcoolique, d'une hydroxylamine de formule R₂₃NH - OH dans laquelle R₂₃ représente un groupement alkyle (C₁-C₆) linéaire ou ramifié, ou cycloalkyle (C₃-C₇), pour conduire au composé de formule (I/e), cas particulier des composés de formule (I) : 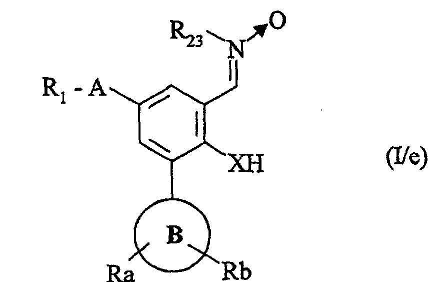 dans laquelle R₁, A, B, Ra, Rb, X et R₂₃ ont la même signification que précédemment,
**c** soit l'action d'un ylure de phosphore de formule R'cCH₂P⁺(C₆H₅)₃Br⁻ (dans laquelle R'c représente un atome d'hydrogène, un groupement alkyle (C₁-C₆) linéaire ou ramifié, cycloalkyle (C₃-C₇), alkoxy (C₁-C₆) linéaire ou ramifié, alkylthio (C₁-C₆) linéaire ou ramifié, phényle substitué ou non, naphtyle substitué ou non, hétérocyclique saturé ou insaturé substitué ou non), en présence de n-butyllithium,
pour conduire au composé de formule (I/f), cas particulier des composés de formule (I) : 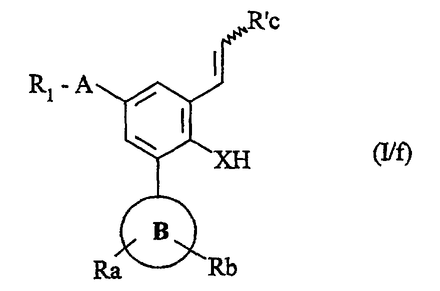 dans laquelle R₁, A, Ra, Rb, B, X et R'c ont la même signification que précédemment,
qui subit, si on le souhaite, une réduction, pour conduire au composé de formule (I/g), cas particulier des composés de formule (I): 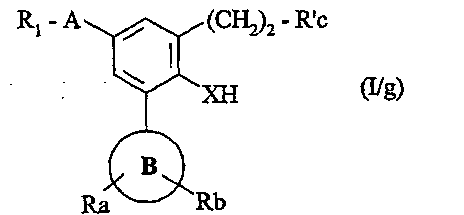 dans laquelle R₁, A, Ra, Rb, B, X et R'c ont la même signification que précédemment,
**d** soit une réaction en milieu oxydant, pour conduire au composé de formule (I/h) : 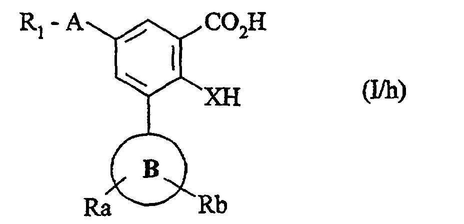 dans laquelle R₁, A, Ra, Rb, B et X ont la même signification que dans la formule (I), composé de formule (I/h) dont on transforme la fonction acide en chlorure d'acide correspondant, qui peut alors être transformé en ester, en amide ou en amine correspondants selon les réactions classiques de la chimie organique,
**e** soit, l'action du chlorhydrate d'hydroxylamine en milieu approprié, pour conduire au composé de formule (I/i), cas particulier des composés de formule (I) : 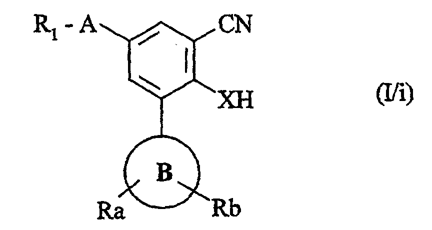 dans laquelle R₁, A, Ra, Rb, B et X ont la même signification que précédemment,
- que l'on fait réagir en milieu alcoolique avec de l'acide chlorhydrique, puis avec une amine Rd-NH-Re (dans laquelle Rd, Re, identiques ou différents, représentent un atome d'hydrogène ou un groupement alkyle (C₁-C₆) linéaire ou ramifié, ou cycloalkyle ou formant ensemble avec l'atome d'azote qui les porte un cycle de 5 à 7 chaînons contenant de 1 à 3 hétéroatomes choisis parmi azote, oxygène et soufre),
pour conduire au composé de formule (I/j), cas particulier des composés de formule (I) : 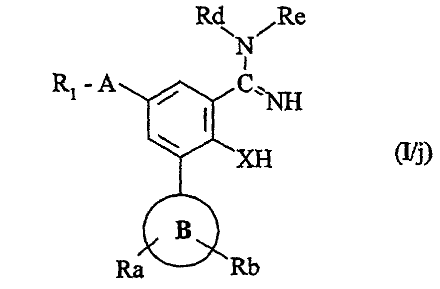 dans laquelle R₁, A, Ra, Rb, X, B, Rd et Re sont tels que définis précédemment,
- ou que l'on fait réagir avec un composé de formule (VI) en milieu basique :
RcO-NH₂ (VI)
dans laquelle Rc est tel que défini dans la formule (I),
pour conduire au composé de formule (I/k), cas particulier des composés de formule (I) : 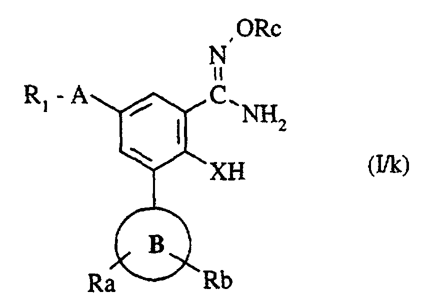 dans laquelle A, B, R₁, Ra, Rb, Rc, et X sont tels que définis précédemment,
**f** soit, enfin avec une hydrazine 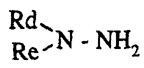 (Rd et Re étant tels que définis précédemment), pour conduire au composé de formule (I/l), cas particulier des composés de formule (I) : 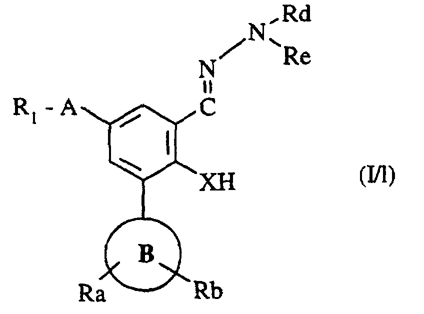 dans laquelle A, B, R₁, Ra, Rb, Rd, Re et X sont tels que définis précédemment, composé de formule (I/a), (I/b), (I/c), (I/d), (I/e), (I/f), (I/g), (I/h), (I/i), (I/l)ou leurs dérivés :
- que l'on soumet éventuellement à l'action d'un dialkylsulfate ou d'un halogénure d'alkyle (C₁-C₆) linéaire ou ramifié ou d'acyle (C₁-C₆) linéaire ou ramifié ou de cycloalkyle (C₃-C₇),
pour conduire au composé de formule (I/m), cas particulier des composés de formule (I): 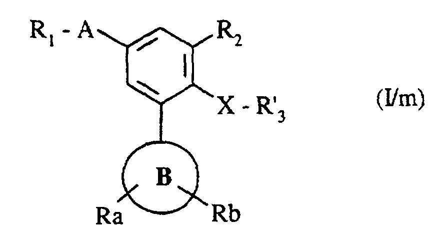 dans laquelle R₁, A, B, Ra, Rb, R₂ et X ont la même signification que précédemment, et R'₃ représente un groupement alkyle (C₁-C₆) linéaire ou ramifié, acyle (C₁-C₆) linéaire ou ramifié ou cycloalkyle (C₃-C₇),
composé de formule (Va) à (I/m) ou leurs dérivés
- qui peut être, le cas échéant, purifié selon une technique classique de purification
- dont on sépare, le cas échéant, les isomères selon une technique classique de séparation,
- que l'on transforme, si on le souhaite, en ses sels d'addition à un acide ou à une base pharmaceutiquement acceptable.

16. Procédé de préparation des composés de formule (I) selon la revendication 1 caractérisé en ce que l'on utilise comme produit de départ un composé de formule (XI) : 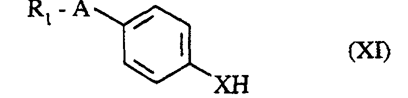 dans laquelle X, A et R₁ ont la même signification que dans la formule (I),
qui subit une réaction de bromation pour conduire au composé de formule (XII) 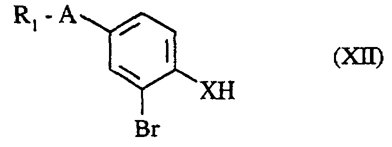 dans laquelle X, A et R₁ ont la même signification que précédemment,
qui est soumis à l'action d'un acide borique de formule (V) : 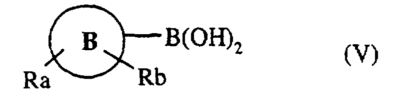 dans laquelle Ra, Rb, et B sont tels que définis dans la formule (I),
pour conduire à un composé de formule (XIII) : 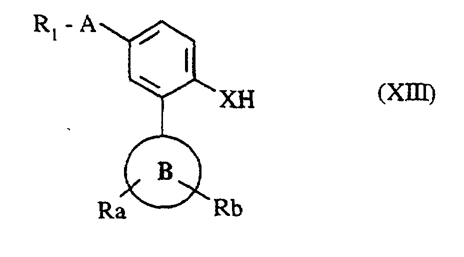 dans laquelle A, R₁, Ra, Rb, B et X sont tels que définis précédemment,
composé (XIII) qui subit une réaction d'halogénation pour conduire au composé de formule (I/n), cas particulier des composés de formule (I): 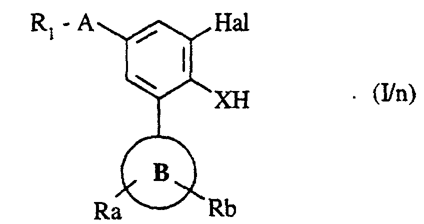 dans laquelle A, R₁, Ra, Rb, B et X sont tels que définis précédemment et Hal représente un halogène,
composé (I/n) qui peut être soumis :
- soit à l'action du cyanure de zinc en présence d'un catalyseur palladié, pour conduire à un composé de formule (I/i) telle que définie précédemment,
- soit à l'action d'un dérivé de l'étain en présence d'un catalyseur palladié, pour conduire à un composé de formule (I/p), cas particulier des composés de formule (I) : 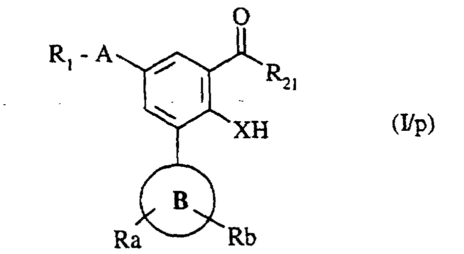 dans laquelle A, R₁, Ra, Rb, B et X sont tels que définis dans la formule (I) et R₂₁, différent d'un atome d'hydrogène a la même définition que dans la formule (I),
composé (I/p) que l'on fait réagir :
- soit avec un composé de formule (VI) :
R₂₂O-NH₂ (VI)
dans laquelle R₂₂ représente un atome d'hydrogène ou un groupement alkyle (C₁-C₆) linéaire ou ramifié, cycloalkyle (C₃-C₇) ou acyle (C₁-C₆) linéaire ou ramifié,
pour conduire au composé de formule (I/q), cas particulier des composés de formule (I) : 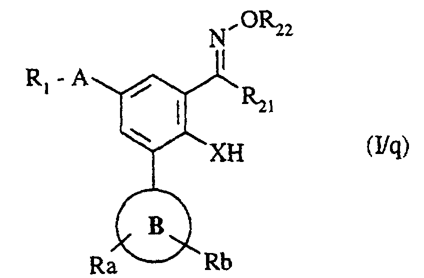 dans laquelle A, B, R₁, Ra, Rb, R₂₁, R₂₂ et X sont tels que définis précédemment ;
- soit avec une hydrazine 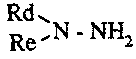 dans laquelle Rd, Re, identiques ou différents, représentent un atome d'hydrogène ou un groupement alkyle (C₁-C₆) linéaire ou ramifié, ou cycloalkyle ou formant ensemble avec l'atome d'azote qui les porte un cycle de 4 à 7 chaînons contenant de 1 à 3 hétéroatomes choisis parmi azote, oxygène et soufre, pour conduire au composé de formule (I/s), cas particulier des composés de formule (I) : 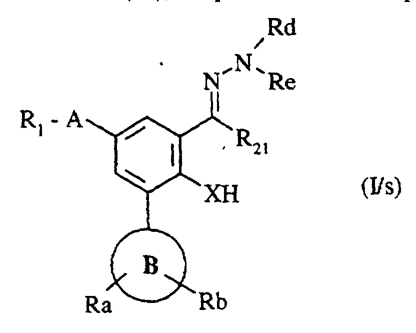 dans laquelle A, B, R₁, Ra, Rb,Rd, Re, R₂₁, et X sont tels que définis précédemment,
composés (I/n), (I/p), (I/q), (I/s) ou leurs dérivés que l'on soumet éventuellement à l'action d'un diallkylsulfate ou d'un halogénure d'alkyle (C₁-C₆) linéaire ou ramifié, ou d'acyle (C₁-C₆) linéaire ou ramifié, ou de cycloalkyle (C₃-C₇), pour conduire au composé de formule (I/m) telle que définie précédemment, composés (I/n) à (I/s) ou leur dérivés :
- qui peuvent être, le cas échéant, purifiés selon une technique classique de purifiaction,
- dont on sépare éventuellement les isomères selon une technique classique de séparation,
- que l'on transforme, si on le souhaite, en leurs sels d'addition à un acide ou à une base pharmaceutiquement acceptable.

17. Compositions pharmaceutiques contenant comme principe actif au moins un composé selon l'une quelconque des revendications 1 à 14, seul ou en combinaison avec un ou plusieurs excipients ou véhicules inertes, non toxiques, pharmaceutiquement acceptables.

18. Compositions pharmaceutiques selon la revendication 17 contenant au moins un principe actif selon l'une quelconque des revendications 1 à 14 utiles comme inhibiteurs de phosphodiestérases du groupe IV.

## Patentansprüche

1. Verbindungen der Formel (I): 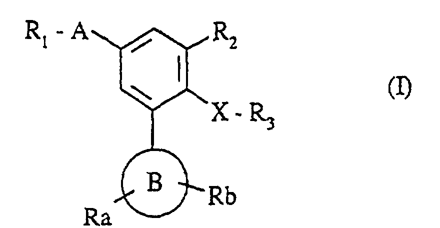 in der:
R₁ eine gegebenenfalls substituierte (C₃-C₇)-Cycloalkylgruppe, eine gegebenenfalls substituierte Phenylgruppe, eine gegebenenfalls substituierte Naphthylgruppe, eine gesättigte oder ungesättigte, gegebenenfalls substituierte, mono- oder bicyclische heterocyclische Gruppe, die 1 bis 3 Heteroatome ausgewählt aus Stickstoff, Sauerstoff oder Schwefel enthält, eine geradkettige oder verzweigte, gegebenenfalls substituierte (C₁-C₆)-Alkylgruppe oder eine geradkettige oder verzweigte, gegebenenfalls substituierte (C₁-C₆)-Alkenylgruppe darstellt;
A eine Bindung (mit der Maßgabe, daß in diesem Fall R₁ von einer C₁- oder C₂-Alkylgruppe verschieden ist), ein Sauerstoffatom, ein Schwefelatom, eine Gruppe der Formel 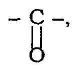 eine Gruppe der Formel 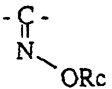 oder eine Gruppe der Formel 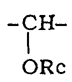 (worin Rc für ein Wasserstoffatom, eine geradkettige oder verzweigte (C₁-C₆)-Alkylgruppe oder eine (C₃-C₇)-Cycloalkylgruppe steht), bedeutet,
R₂ ein Halogenatom, eine geradkettige oder verzweigte, gegebenenfalls substituierte (C₁-C₆)-Alkylgruppe, eine geradkettige oder verzweigte, gegebenenfalls substituierte (C₁-C₆)-Alkenylgruppe, eine geradkettige oder verzweigte, gegebenenfalls substituierte (C₁-C₆)-Alkinylgruppe, eine gegebenenfalls substituierte (C₃-C₇)-Cycloalkylgruppe, eine Formylgruppe, eine Carboxygruppe, eine geradkettige oder verzweigte (C₁-C₆)-Alkylcarbonylgruppe, eine (C₃-C₇)-Cycloalkylcarbonylgruppe, eine geradkettige oder verzweigte (C₁-C₆)-Alkoxycarbonylgruppe, eine (C₃-C₇)-Cycloalkyloxycarbonylgruppe, eine Carbamoylgruppe (die gegebenenfalls durch ein oder zwei geradkettige oder verzweigte (C₁-C₆)-Alkylgruppen, (C₃-C₇)-Cycloalkylgruppen, Hydroxygruppen, geradkettige oder verzweigte (C₁-C₆)-Alkoxygruppen, (C₃-C₇)-Cycloalkyloxygruppen substituiert ist oder die zusammen mit dem sie tragenden Stickstoffatom einen Ring mit 5 bis 7 Kettengliedern, der 1 bis 3 Heteroatome ausgewählt aus Stickstoff, Sauerstoff oder Schwefel enthält, bilden, eine Aminogruppe (die gegebenenfalls durch eine oder zwei geradkettige oder verzweigte (C₁-C₆)-Alkylgruppen, geradkettige oder verzweigte (C₁-C₆)-Acylgruppen, (C₃-C₇)-Cycloalkylgruppen substituiert ist oder die zusammen mit dem sie tragenden Stickstoffatom einen Ring mit 5 bis 7 Kettengliedern, der 1 bis 3 Heteroatome ausgewählt aus Stickstoff, Sauerstoff oder Schwefel enthält, bilden), eine Formamidogruppe, eine Cyanogruppe, eine Amidinogruppe (die gegebenenfalls durch eine oder zwei geradkettige oder verzweigte (C₁-C₆)-Alkylgruppen, (C₃-C₇)-Cycloalkylgruppen substituiert ist oder die zusammen mit dem sie tragenden Stickstoffatom einen Ring mit 5 bis 7 Kettengliedern, der 1 bis 3 Heteroatome ausgewählt aus Stickstoff, Sauerstoff und Schwefel enthält, bilden), eine Hydroxyaminomethylgruppe (die gegebenenfalls unabhängig voneinander am Stickstoffatom und am Sauerstoffatom durch eine geradkettige oder verzweigte (C₁-C₆)-Alkylgruppe, eine (C₃-C₇)-Cycloalkylgruppe, eine geradkettige oder verzweigte (C₁-C₆)-Acylgruppe substituiert ist), eine Amid-oximgruppe (die gegebenenfalls unabhängig voneinander am Stickstoffatom oder am Sauerstoffatom durch eine geradkettige oder verzweigte (C₁-C₆)-Alkylgruppe, eine (C₃-C₇)-Cycloalkylgruppe substituiert ist oder zusammen mit dem sie tragenden Stickstoffatom einen Ring mit 5 bis 7 Kettengliedern bildet, der 1 bis 3 Heteroatome ausgewählt aus Stickstoff, Sauerstoff und Schwefel enthält), eine Hydrazonogruppe (die gegebenenfalls durch eine oder zwei geradkettige oder verzweigte (C₁-C₆)-Alkylgruppen, (C₃-C₇)-Cycloalkylgruppen substituiert ist oder die zusammen mit dem sie tragenden Stickstoffatom einen Ring mit 5 bis 7 Kettengliedern bilden, der 1 bis 3 Heteroatome ausgewählt aus Stickstoff, Sauerstoff und Schwefel enthält), oder eine Gruppe ausgewählt aus: 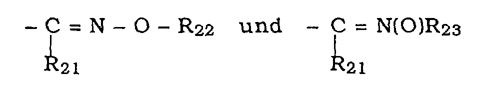 worin R₂₁ ein Wasserstoffatom, eine geradkettige oder verzweigte (C₁-C₆)-Alkylgruppe oder eine (C₃-C₇)-Cycloalkylgruppe,
R₂₂ ein Wasserstoffatom oder eine geradkettige oder verzweigte (C₁-C₆)-Alkylgruppe, eine (C₃-C₇)-Cycloalkylgruppe oder eine geradkettige oder verzweigte (C₁-C₆)-Acylgruppe und
R₂₃ eine geradkettige oder verzweigte (C₁-C₆)-Alkylgruppe oder eine (C₃-C₇)-Cycloalkylgruppe darstellen, bedeutet,
R₃ ein Wasserstoffatom, eine geradkettige oder verzweigte (C₁-C₆)-Alkylgruppe, eine (C₃-C₇)-Cycloalkylgruppe oder eine geradkettige oder verzweigte (C₁-C₆)-Acylgruppe darstellt, oder 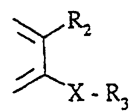 zusammen den Ring 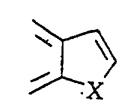 bilden,
X ein Sauerstoffatom, ein Schwefelatom oder eine Gruppe NR" darstellt (worin R" ein Wasserstoffatom, eine geradkettige oder verzweigte (C₁-C₆)-Alkylgruppe, eine (C₃-C₇)-Cycloalkylgruppe oder eine geradkettige oder verzweigte (C₁-C₆)-Acylgruppe bedeutet),
wobei der Ring B einen Phenyl- oder Pyridylring darstellt,
Ra, Rb, die gleichartig oder verschieden sind, Wasserstoffatome, Halogenatome, geradkettige oder verzweigte (C₁-C₆)-Alkylgruppen, Hydroxygruppen, geradkettige oder verzweigte (C₁-C₆)-Alkoxygruppen, Carboxygruppen, geradkettige oder verzweigte (C₁-C₆)-Polyhalogenalkylgruppen, Cyanogruppen, Nitrogruppen, geradkettige oder verzweigte (C₁-C₆)-Alkoxycarbonylgruppen, (C₃-C₇)-Cycloalkyloxycarbonylgruppen, Aminogruppen (die gegebenenfalls durch eine oder zwei geradkettige oder verzweigte (C₁-C₆)-Alkylgruppen, geradkettige oder verzweigte (C₁-C₆)-Acylgruppen oder (C₃-C₇)-Cycloalkylgruppen substituiert sind), Carbamoylgruppen (die gegebenenfalls durch eine oder zwei geradkettige oder verzweigte (C₁-C₆)-Alkylgruppen, (C₃-C₇)-Cycloalkylgruppen oder geradkettige oder verzweigte (C₁-C₆)-Alkoxygruppen substituiert sind), Sulfogruppen, geradkettige oder verzweigte (C₁-C₆)-Alkylsulfonylgruppen, (C₃-C₇)Cycloalkylsulfonylgruppen oder Aminosulfonylgruppen (die gegebenenfalls durch eine oder zwei geradkettige oder verzweigte (C₁-C₆)-Alkylgruppen oder (C₃-C₇)-Cycloalkylgruppen substituiert sind) bedeuten,
mit der Maßgabe, daß
- wenn A eine Bindung oder ein Sauerstoffatom, B einen Phenylring, R₁ eine gegebenenfalls substituierte (C₃-C₇)-Cycloalkylgruppe, eine gegebenenfalls substituierte Phenylgruppe oder eine gegebenenfalls substituierte Naphthylgruppe, X ein Sauerstoffatom oder eine Gruppe NR" (worin R" ein Wasserstoffatom oder eine geradkettige oder verzweigte (C₁-C₆)-Alkylgruppe darstellt) und R₃ ein Wasserstoffatom oder eine geradkettige oder verzweigte (C₁-C₆)-Alkylgruppe bedeuten, R₂ von einer Carboxygruppe verschieden ist,
- unter einer substituierten Alkylgruppe, substituierten Alkenylgruppe, substituierten Alkinylgruppe oder substituierte Cycloalkylgruppe eine Substitution durch ein oder mehrere Halogenatome oder geradkettige oder verzweigte (C₁-C₆)-Alkoxygruppen, geradkettige oder verzweigte (C₁-C₆)-Alkylthiogruppen, (C₃-C₇)-Cycloalkylgruppen, gegebenenfalls substituierte Phenylgruppen, gegebenenfalls substituierte Naphthylgruppen oder gegebenenfalls substituierte Heterocyclen (wie beispielsweise eine Pyridylgruppe) zu verstehen ist, wobei eine bevorzugte gesättigte oder ungesättigte Alkylgruppe die gegebenenfalls substituierte Benzylgruppe ist,
- unter einer substituierten Phenylgruppe, substituierten Naphthylgruppe, substituierten heterocyclischen Gruppe oder substituierten Benzylgruppe eine Substitution durch ein oder mehrere Halogenatome oder geradkettige oder verzweigte (C₁-C₆)-Alkylgruppen, geradkettige oder verzweigte (C₁-C₆)-Alkoxygruppen oder Trihalogenmethylgruppen zu verstehen ist,
deren Enantiomere, Diastereoisomere sowie deren Additionssalze mit einer pharmazeutisch annehmbaren Säure oder Base.

2. Verbindungen der Formel (I) nach Anspruch 1, worin der Ring B einen Phenylring bedeutet.

3. Verbindungen der Formel (I) nach Anspruch 1, worin der Ring B einen Pyridylring bedeutet.

4. Verbindungen der Formel (I) nach Anspruch 1, worin X ein Sauerstoffatom bedeutet.

5. Verbindungen der Formel (I) nach Anspruch 1, worin R₁ eine Alkylgruppe bedeutet, die durch eine gegebenenfalls substituierte Phenylgruppe substituiert ist.

6. Verbindungen der Formel (I) nach Anspruch 1, worin R₁ eine Alkylgruppe bedeutet, die durch eine gesättigte oder ungesättigte, gegebenenfalls substituierte heterocyclische Gruppe, die 1 bis 3 Heteroatome ausgewählt aus Sauerstoff, Stickstoff oder Schwefel enthält, substituiert ist.

7. Verbindungen der Formel (I) nach Anspruch 6, worin R₁ eine Alkylgruppe bedeutet, die durch eine gegebenenfalls substituierte Pyridylgruppe substituiert ist.

8. Verbindungen der Formel (I) nach Anspruch 6, worin R₁ eine gesättigte oder ungesättigte, gegebenenfalls substituierte heterocyclische Gruppe, die 1 bis 3 Heteroatome ausgewählt aus Sauerstoff, Stickstoff oder Schwefel enthält, bedeutet.

9. Verbindungen der Formel (I) nach Anspruch 1, worin R2 ein Halogenatom, eine geradkettige oder verzweigte, gegebenenfalls substituierte (C₁-C₆)-Alkylgruppe, eine geradkettige oder verzweigte, gegebenenfalls substituierte (C₁-C₆)-Alkenylgruppe, eine geradkettige oder verzweigte, gegebenenfalls substituierte (C₁-C₆)-Alkinylgruppe, eine gegebenenfalls substituierte (C₃-C₇)-Cycloalkylgruppe, eine Formylgruppe, eine Carboxygruppe, eine geradkettige oder verzweigte (C₁-C₆)-Alkylcarbonylgruppe, eine (C₃-C₇)-Cycloalkylcarbonylgruppe, eine geradkettige oder verzweigte (C₁-C₆)-Alkoxycarbonylgruppe, eine (C₃-C₇)-Cycloalkyloxycar-bonylgruppe, eine Carbamoylgruppe (die gegebenenfalls durch eine oder zwei geradkettige oder verzweigte (C₁-C₆)-Alkylgruppen, (C₃-C₇)-Cycloalkylgruppen, Hydroxygruppen, geradkettige oder verzweigte (C₁-C₆)-Alkoxygruppen oder (C₃-C₇)-Cycloalkyloxygruppen substituiert ist oder die zusammen mit dem sie tragenden Stickstoffatom einen Ring mit 5 bis 7 Kettengliedern bilden, der 1 bis 3 Heteroatome ausgewählt aus Stickstoff, Sauerstoff und Schwefel enthält), eine Aminogruppe (die gegebenenfalls durch eine oder zwei geradkettige oder verzweigte (C₁-C₆)-Alkylgruppen, geradkettige oder verzweigte (C₁-C₆)-Acylgruppen oder (C₃-C₇)-Cycloalkylgruppen substituiert ist oder die zusammen mit dem sie tragenden Stickstoffatom einen Ring mit 5 bis 7 Kettengliedern bilden, der 1 bis 3 Heteroatome ausgewählt aus Stickstoff, Sauerstoff und Schwefel enthält), eine Formamidogruppe, eine Cyanogruppe, eine Amidinogruppe (die gegebenenfalls durch eine oder zwei geradkettige oder verzweigte (C₁-C₆)-Alkylgruppen oder (C₃-C₇)-Cycloalkylgruppen substituiert ist oder die zusammen mit dem sie tragenden Stickstoffatom einen Ring mit 5 bis 7 Kettengliedern bilden, der 1 bis 3 Heteroatome ausgewählt aus Stickstoff, Sauerstoff und Schwefel enthält), eine Hydroxyaminomethylgruppe (die gegebenenfalls unabhängig voneinander am Stickstoffatom oder am Sauerstoffatom durch eine geradkettige oder verzweigte (C₁-C₆)-Alkylgruppe, eine (C₃-C₇)-Cycloalkylgruppe oder eine geradkettige oder verzweigte (C₁-C₆)-Acylgruppe substituiert ist), eine Amidoximgruppe (die gegebenenfalls unabhängig voneinander am Stickstoffatom oder am Sauerstoffatom durch eine geradkettige oder verzweigte (C₁-C₆)-Alkylgruppe oder eine (C₃-C₇)-Cycloalkylgruppe substituiert ist oder die zusammen mit dem sie tragenden Stickstoffatom einen Ring mit 5 bis 7 Kettengliedern bilden, der 1 bis 3 Heteroatome ausgewählt aus Stickstoff, Sauerstoff und Schwefel enthält), eine Hydrazonogruppe (die gegebenenfalls durch eine oder zwei geradkettige oder verzweigte (C₁-C₆)-Alkylgruppen oder (C₃-C₇)-Cycloalkylgruppen substituiert ist oder die zusammen mit dem sie tragenden Stickstoffatom einen Ring mit 5 bis 7 Kettengliedern bilden, der 1 bis 3 Heteroatome ausgewählt aus Stickstoff, Sauerstoff und Schwefel enthält), oder eine Gruppe ausgewählt aus: 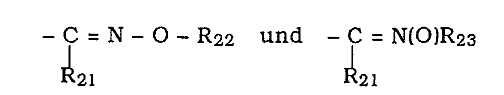 in denen R₂₁ ein Wasserstoffatom, eine geradkettige oder verzweigte (C₁-C₆)-Alkylgruppe oder eine (C₃-C₇)-Cycloalkylgruppe,
R₂₂ ein Wasserstoffatom oder eine geradkettige oder verzweigte (C₁-C₆)-Alkylgruppe, eine (C₃-C₇)-Cycloalkylgruppe oder eine geradkettige oder verzweigte (C₁-C₆)-Acylgruppe und
R₂₃ eine geradkettige oder verzweigte (C₁-C₆)-Alkylgruppe oder eine (C₃-C₇)-Cycloalkylgruppe bedeuten, darstellt.

10. Verbindungen der Formel (I) nach Anspruch 1, worin 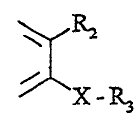 zusammen den Ring 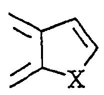 bildet.

11. Verbindungen der Formel (I) nach Anspruch 10, worin X ein Sauerstoffatom bedeutet.

12. Verbindung der Formel (I) nach Anspruch 1, nämlich 2-Hydroxy-3-(3-nitrophenyl)-5-pyridylbenzaldehyd-oxim,
dessen Isomeren sowie dessen Additionssalze mit einer pharmazeutisch annehmbaren Säure oder Base.

13. Verbindung der Formel (I) nach Anspruch 1, nämlich 5-Benzyl-2-hydroxy-3-(3-nitrophenyl)-benzaldehyd-oxim,
dessen Isomeren sowie dessen Additionssalze mit einer pharmazeutisch annehmbaren Base.

14. Verbindung der Formel (I) nach Anspruch 1, nämlich 7-(3-Nitrophenyl)-5-(4-pyridylmethyl)-benzo[b]furan,
dessen Isomeren sowie dessen Additionssalze mit einer pharmazeutisch annehmbaren Säure.

15. Verfahren zur Herstellung der Verbindungen der Formel (I) nach Anspruch 1, dadurch gekennzeichnet, daß man als Ausgangsprodukt eine Verbindung der Formel (II) verwendet: 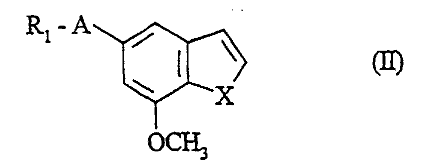 in der R₁, A und X die bezüglich der Formel (I) angegebenen Bedeutungen besitzen,
welche man der Einwirkung von Bortribromid in Dichlormethanmedium unterwirft zur Bildung der Verbindung der Formel (III): 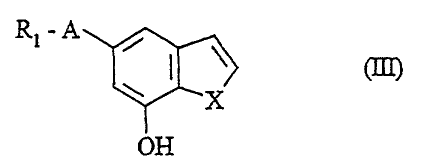 in der R₁, A und X die bezüglich der Formel (I) angegebenen Bedeutungen besitzen,
welche man der Einwirkung von Trifluormethansulfonsäureanhydrid in Pyridin unterwirft zur Bildung der Verbindung der Formel (IV): 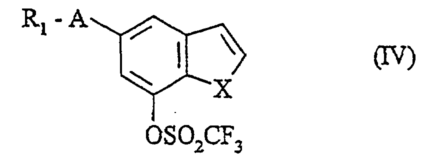 in der R₁, A und X die bezüglich der Formel (I) angegebenen Bedeutungen besitzen,
welche man der Einwirkung einer Borsäure der Formel (V) unterwirft: 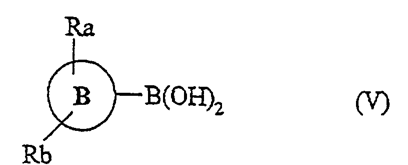 in der B, Ra und Rb die bezüglich der Formel (I) angegebenen Bedeutungen besitzen,
zur Bildung der Verbindung der Formel (I/a), einem Sonderfall der Verbindungen der Formel (I): 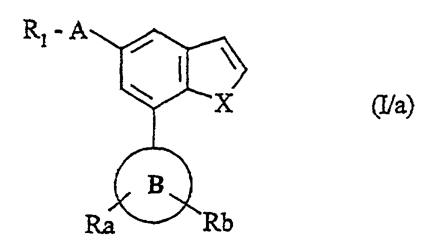 in der R₁, A, Ra, Rb, B und X die bezüglich der Formel (I) angegebenen Bedeutungen besitzen,
welche Verbindung der Formel (I/a) man gewünschtenfalls einer oxidierenden Spaltung unterwirft zur Bildung der Verbindung der Formel (I/b), einem Sonderfall der Verbindungen der Formel (I): 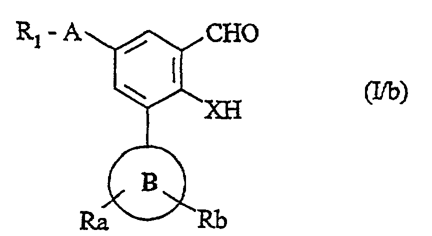 in der R₁, A, Ra, Rb, B und X die bezüglich der Formel (I) angegebenen Bedeutungen besitzen,
welche Verbindung der Formel (I/b) man:
**a** entweder der Einwirkung der Verbindung der Formel (VI) in basischem Medium unterwerfen kann:
R₂₂O - NH₂ (VI)
in der R₂₂ ein Wasserstoffatom oder eine geradkettige oder verzweigte (C₁-C₆)-Alkylgruppe, eine (C₃-C₇)-Cycloalkylgruppe oder eine geradkettige oder verzweigte (C₁-C₆)-Acylgruppe darstellt,
zur Bildung der Verbindung der Formel (I/c), einem Sonderfall der Verbindungen der Formel (I): 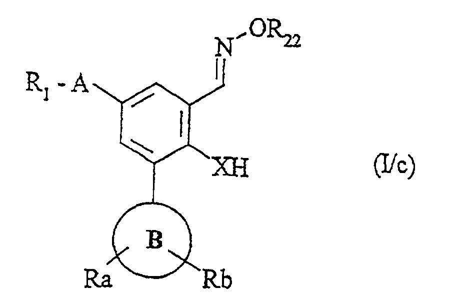 in der R₁, A, Ra, Rb, B, X und R₂₂ die oben angegebenen Bedeutungen besitzen, welche Verbindung der Formel (I/c) man gegebenenfalls dann, wenn R₂₂ von einer Acylgruppe verschieden ist, in saurem Medium in eine Verbindung der Formel (I/d) umwandelt, einem Sonderfall der Verbindungen der Formel (I): 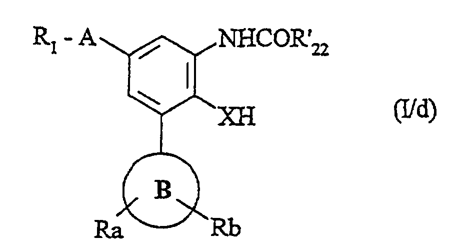 in der R₁, A, Ra, Rb, B und X die oben angegebenen Bedeutungen besitzen und R'₂₂ von einer Acylgruppe verschieden ist und sonst die gleichen Bedeutungen besitzt wie R₂₂,
**b** oder der Einwirkung eines Hydroxylamins der Formel R₂₃NH-OH, in der R23 eine geradkettige oder verzweigte (C₁-C₆)-Alkylgruppe oder eine (C₃-C₇)-Cycloalkylgruppe darstellt, in alkoholischem Medium unterwirft zur Bildung der Verbindung der Formel (I/e), einem Sonderfall der Verbindungen der Forme (I): 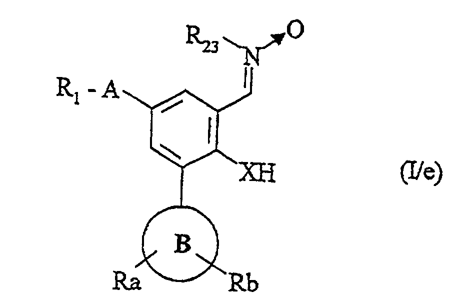 in der R₁, A, B, Ra, Rb, X und R₂₃ die oben angegebenen Bedeutungen besitzen,
**c** oder der Einwirkung eines Phosphorylids der Formel R'cCH₂P⁺(C₆H₅)₃Br⁻ (worin R'c ein Wasserstoffatom, eine geradkettige oder verzweigte (C₁-C₆)-Alkylgruppe, eine (C₃-C₇)-Cycloalkylgruppe, eine geradkettige oder verzweigte (C₁-C₆)-Alkoxygruppe, eine geradkettige oder verzweigte (C₁-C₆)-Alkylgruppe, eine gegebenenfalls substituierte Phenylgruppe, eine gegebenenfalls substituierte Naphthylgruppe, eine gesättigte oder ungesättigte, gegebenenfalls substituierte heterocyclische Gruppe darstellt) in Gegenwart von n-Butyllithium unterwirft zur Bildung der Verbindung der Formel (I/f), einem Sonderfall der Verbindungen der Formel (I): 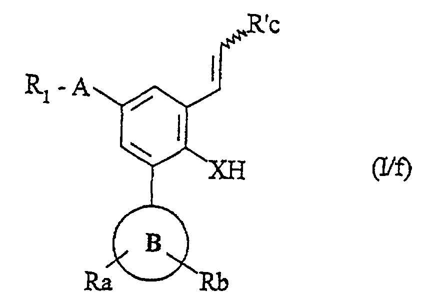 in der R₁, A, Ra, Rb, B, X und R'c die oben angegebenen Bedeutungen besitzen, welche man gewünschtenfalls einer Reduktion unterzieht zur Bildung der Verbindung der Formel (I/g), einem Sonderfall der Verbindungen der Formel (I): 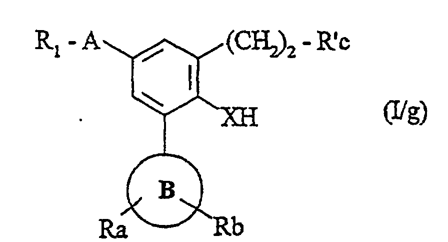 in der R₁, A, Ra, Rb, B, X und R'c die oben angegebenen Bedeutungen besitzen,
**d** oder einer Reaktion in oxidierendem Medium unterwirft zur Bildung der Verbindung der Formel (I/h): 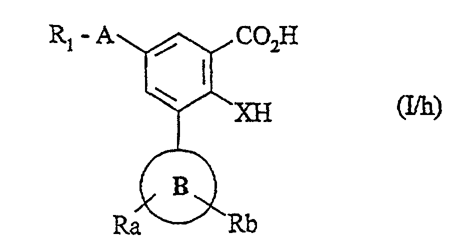 in der R₁, A, Ra, Rb, B und X die bezüglich der Formel (I) angegebenen Bedeutungen besitzen,
wonach man die Säurefunktion der Verbindung der Formel (I/h) in das entsprechende Säurechlorid umwandelt, welches dann mit Hilfe klassischer Reaktionen der organischen Chemie in entsprechende Ester, Amide oder Amine umgewandelt werden kann,
**e** oder der Einwirkung von Hydroxylamin-Hydrochlorid in einem geeigneten Medium unterwirft,
zur Bildung der Verbindung der Formel (I/i), einem Sonderfall der Verbindungen der Formel (I): 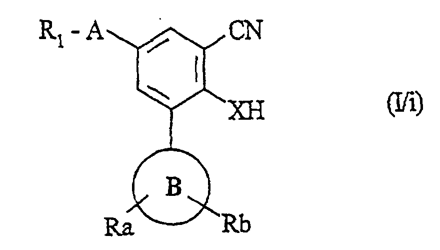 in der R₁, A, Ra, Rb, B und X die oben angegebenen Bedeutungen besitzen,
- welche man in alkoholischem Medium mit Chlorwasserstoffsäure und dann mit einem Amin Rd-NH-Re (worin Rd und Re, die gleichartig oder verschieden sind, Wasserstoffatome oder geradkettige oder verzweigte (C₁-C₆)-Alkylgruppen oder Cycloalkylgruppen bedeuten oder zusammen mit dem sie tragenden Stickstoffatom einen Ring mit 5 bis 7 Kettengliedern bilden, der 1 bis 3 Heteroatome ausgewählt aus Stickstoff, Sauerstoff und Schwefel enthält) umsetzt, zur Bildung der Verbindung der Formel (I/j), einem Sonderfall der Verbindungen der Formel (I): 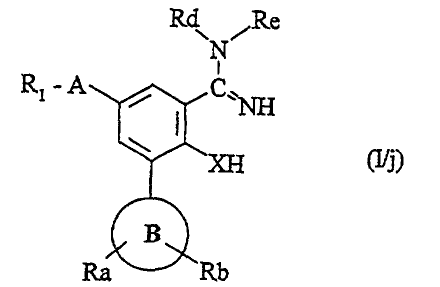 in der R₁, A, Ra, Rb, X, B, Rd und Re die oben angegebenen Bedeutungen besitzen,
- oder welche man in basischem Medium mit einer Verbindung der Formel (VI) umsetzt:
RcO - NH₂ (VI)
in der Rc die bezüglich der Formel (I) angegebenen Bedeutungen besitzt, zur Bildung der Verbindung der Formel (I/k), einem Sonderfall der Verbindungen der Formel (I): 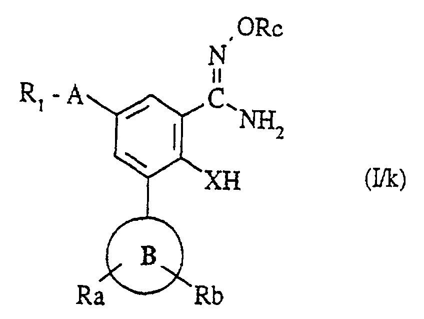 in der A, B, R₁, Ra, Rb, Rc und X die oben angegebenen Bedeutungen besitzen,
**f** oder schließlich mit einem Hydrazin 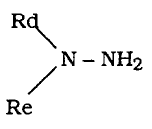 (worin Rd und Re die oben angegebenen Bedeutungen besitzen) umsetzt zur Bildung der Verbindung der Formel (I/1), einem Sonderfall der Verbindungen der Formel (I): 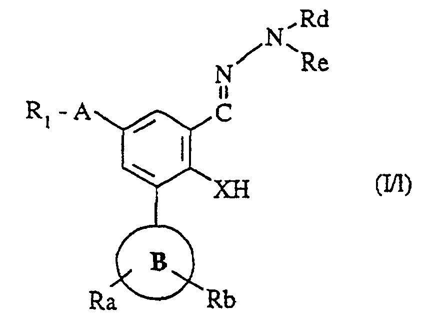 in der A, B, R₁, Ra, Rb, Rd, Re und X die oben angegebenen Bedeutungen besitzen,
welche Verbindung der Formeln (I/a), (I/b), (I/c), (I/d), (I/e), (I/f), (I/g), (I/h), (I/i), (I/l) oder deren Derivate:
- man gegebenenfalls der Einwirkung eines Dialkylsulfats oder eines geradkettigen oder verzweigten (C₁-C₆)-Alkylhalogenids oder eines geradkettige oder verzweigte (C₁-C₆)-Acylhalogenids oder eines C₃-C₇)-Cycloalkylhalogenids unterwirft
zur Bildung der Verbindung der Formel (I/m), einem Sonderfall der Verbindungen der Formel (I): 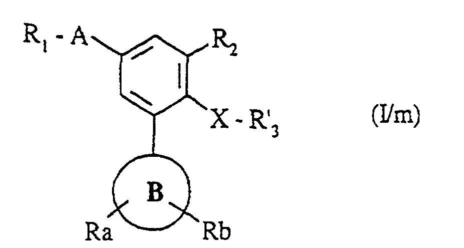 in der R₁, A, B, Ra, Rb, R₂ und X die oben angegebenen Bedeutungen besitzen und R'₃ eine geradkettige oder verzweigte (C₁-C₆)-Alkylgruppe, eine geradkettige oder verzweigte (C₁-C₆)-Acylgruppe oder eine (C₃-C₇)-Cycloalkylgruppe bedeutet, welche Verbindungen der Formeln (I/a) bis (I/m) oder deren Derivate man:
- gegebenenfalls mit Hilfe einer klassischen Reinigungsmethode reinigen kann,
- gegebenenfalls mit Hilfe einer klassischen Trennmethode in ihre Isomeren trennen kann, und
- gewünschtenfalls in ihre Additionssalze mit einer pharmazeutisch annehmbaren Säure oder Base umwandeln kann.

16. Verfahren zur Herstellung der Verbindungen der Formel (I) nach Anspruch 1, dadurch gekennzeichnet, daß man als Ausgangsprodukt eine Verbindung der Formel (XI) verwendet: 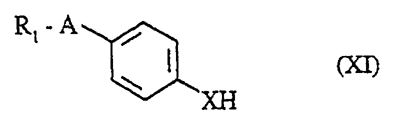 in der X, A und R₁ die bezüglich der Formel (I) angegebenen Bedeutungen besitzen,
welche man einer Bromierungsreaktion unterwirft zur Bildung der Verbindung der Formel (XII) 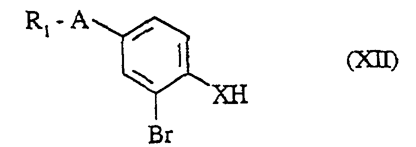 in der X, A und R₁ die oben angegebenen Bedeutungen besitzen,
welche man der Einwirkung einer Borsäure der Formel (V) unterwirft: 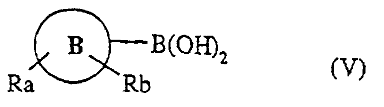 in der Ra, Rb und B die bezüglich der Formel (I) angegebenen Bedeutungen besitzen, zur Bildung einer Verbindung der Formel (XIII): 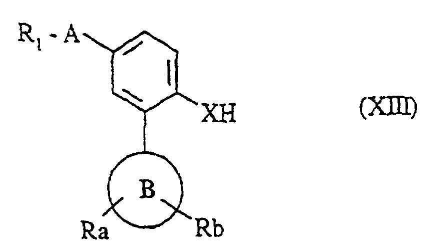 in der A, R₁, Ra, Rb, B und X die oben angegebenen Bedeutungen besitzen,
welche Verbindung (XIII) man einer Halogenierungsreaktion unterwirft zur Bildung der Verbindung der Formel (I/n), einem Sonderfall der Verbindungen der Formel (I): 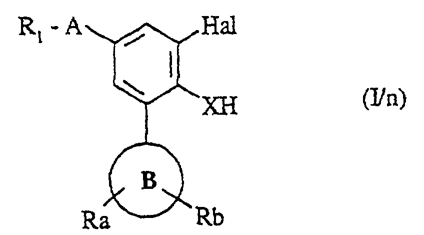 in der A, R₁, Ra, Rb, B und X die oben angegebenen Bedeutungen besitzen und Hal ein Halogen bedeutet,
welche Verbindung (I/n):
- entweder der Einwirkung von Zinkcyanid in Gegenwart eines Palladiumkatalysators unterworfen werden kann zur Bildung einer Verbindung der Formel (I/i), wie sie oben definiert worden ist,
- oder der Einwirkung eines Zinnderivats in Gegenwart eines Palladiumkatalysators unterworfen werden kann zur Bildung einer Verbindung der Formel (I/p), einem Sonderfall der Verbindungen der Formel (I): 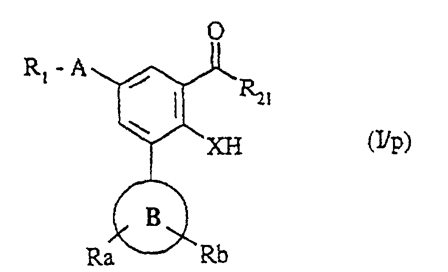 in der A, R₁, Ra, Rb, B und X die bezüglich der Formel (I) angegebenen Bedeutungen besitzen und R₂₁, welches von einem Wasserstoffatom verschieden ist, die bezüglich der Formel (I) angegebenen Bedeutungen besitzt, welche Verbindung (I/p) man:
- entweder mit einer Verbindung der Formel (VI) umsetzt:
R₂₂O-NH₂ (VI)
in der R22 ein Wasserstoffatom oder eine geradkettige oder verzweigte (C₁-C₆)-Alkylgruppe, eine (C₃-C₇)-Cycloalkylgruppe oder eine geradkettige oder verzweigte (C₁-C₆)-Acylgruppe bedeutet,
zur Bildung der Verbindung der Formel (I/q), einem Sonderfall der Verbindungen der Formel (I): 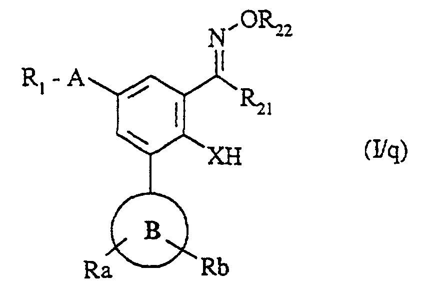 in der A, B, R₁, Ra, Rb, R₂₁, R₂₂ und X die oben angegebenen Bedeutungen besitzen;
- oder mit einem Hydrazin 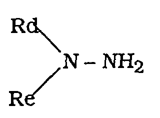 in der Rd und Re, die gleichartig oder verschieden sind, ein Wasserstoffatom oder geradkettige oder verzweigte (C₁-C₆)-Alkylgruppen oder Cycloalkylgruppen bedeuten oder gemeinsam mit dem sie tragenden Stickstoffatom einen Ring mit 4 bis 7 Kettengliedern bilden, der 1 bis 3 Heteroatome ausgewählt aus Stickstoff, Sauerstoff und Schwefel enthält,
umsetzt zur Bildung der Verbindung der Formel (I/s), einem Sonderfall der Verbindungen der Formel (I): 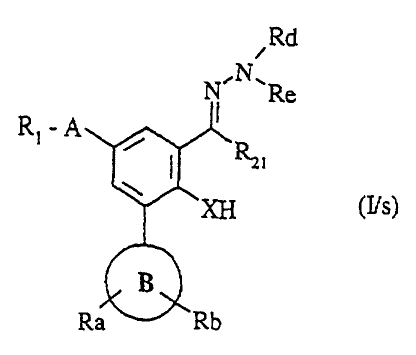 in der A, B, R₁, Ra, Rb, Rd, Re, R₂₁ und X die oben angegebenen Bedeutungen besitzen,
welche Verbindungen (I/n), (I/p), (I/q) und (I/s) oder deren Derivate man gegebenenfalls der Einwirkung eines Dialkylsulfats oder eines geradkettigen oder verzweigten (C₁-C₆)-Alkylhalogenids oder eines geradkettigen oder verzweigten (C₁-C₆)-Acylhalogenids oder eines (C₃-C₇)-Cycloalkylhalogenids unterwirft zur Bildung der Verbindung der Formel (I/m), wie sie oben definiert worden ist, welche Verbindungen (I/n) bis (I/s) oder deren Derivate:
- gegebenenfalls mit Hilfe einer klassischen Reinigungsmethode gereinigt werden können;
- gegebenenfalls mit Hilfe einer klassischen Trennmethode in ihre Isomeren aufgetrennt werden können,
- gewünschtenfalls in ihre Additionssalze mit einer pharmazeutisch annehmbaren Säure oder Base umgewandelt werden können.

17. Pharmazeutische Zubereitungen enthaltend als Wirkstoff mindestens eine Verbindung nach einem der Ansprüche 1 bis 14 allein oder in Kombination mit einem oder mehreren inerten, nichttoxischen, pharmazeutisch annehmbaren Trägermaterialien oder Bindemitteln.

18. Pharmazeutische Zubereitungen nach Anspruch 17 enthaltend mindestens einen Wirkstoff nach einem der Ansprüche 1 bis 14 als Inhibitoren von Phosphodiesterasen der Gruppe IV.

## Claims

1. Compound of formula (I) 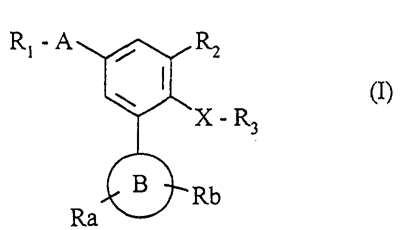 wherein:
R₁ represents a substituted or unsubstituted (C₃-C₇)-cycloalkyl group, a substituted or unsubstituted phenyl group, a substituted or unsubstituted naphthyl group, a saturated or unsaturated, substituted or unsubstituted, mono- or bi-cyclic heterocyclic group containing from 1 to 3 hetero atoms selected from nitrogen, oxygen and sulphur, a linear or branched, substituted or unsubstituted (C₁-C₆)-alkyl group or a linear or branched, substituted or unsubstituted (C₁-C₆)-alkenyl group;
A represents a bond (with the proviso that in that case R₁ is other than a C₁- or C₂-alkyl group), an oxygen atom, a sulphur atom, a group 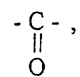 a group 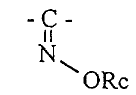 or a group 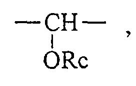 (wherein Rc represents a hydrogen atom, a linear or branched (C₁-C₆)-alkyl group or a (C₃-C₇)-cycloalkyl group),
R₂ represents a halogen atom, a linear or branched, substituted or unsubstituted (C₁-C₆)-alkyl group, a linear or branched, substituted or unsubstituted (C₁-C₆)-alkenyl group, a linear or branched, substituted or unsubstituted (C₁-C₆)-alkynyl group, a substituted or unsubstituted (C₃-C₇)-cycloalkyl group, a formyl group, a carboxy group, a linear or branched (C₁-C₆)-alkylcarbonyl group, a (C₃-C₇)-cycloalkylcarbonyl group, a linear or branched (C₁-C₆)-alkoxycarbonyl group, a (C₃-C₇)-cycloalkyloxycarbonyl group, a carbamoyl group (optionally substituted by one or two groups linear or branched (C₁-C₆)-alkyl, (C₃-C₇)-cycloalkyl, hydroxy, linear or branched (C₁-C₆)-alkoxy, (C₃-C₇)-cycloalkyloxy, or forming together with the nitrogen atom carrying them a ring having from 5 to 7 ring members and containing from 1 to 3 hetero atoms selected from nitrogen, oxygen and sulphur), an amino group (optionally substituted by one or two groups linear or branched (C₁-C₆)-alkyl, linear or branched (C₁-C₆)-acyl, (C₃-C₇)-cycloalkyl, or forming together with the nitrogen atom carrying them a ring having from 5 to 7 ring members and containing from 1 to 3 hetero atoms selected from nitrogen, oxygen and sulphur), a formamido group, a cyano group, an amidino group (optionally substituted by one or two groups linear or branched (C₁-C₆)-alkyl, (C₃-C₇)-cycloalkyl, or forming together with the nitrogen carrying them a ring having from 5 to 7 ring members and containing from 1 to 3 hetero atoms selected from nitrogen, oxygen and sulphur), a hydroxyaminomethyl group (optionally substituted, independently on the nitrogen or oxygen atom, by a linear or branched (C₁-C₆)-alkyl group, a (C₃-C₇)-cycloalkyl group, a linear or branched (C₁-C₆)-acyl group), an amide oxime group (optionally substituted, independently on the nitrogen or oxygen atom, by a linear or branched (C₁-C₆)-alkyl group, a (C₃-C₇)-cycloalkyl group, or forming with the nitrogen atom carrying them a ring having from 5 to 7 ring members and containing from 1 to 3 hetero atoms selected from nitrogen, oxygen and sulphur), a hydrazono group (optionally substituted by one or two groups linear or branched (C₁-C₆)-alkyl, (C₃-C₇)-cycloalkyl, or forming with the nitrogen atom carrying them a ring having from 5 to 7 ring members and containing from 1 to 3 hetero atoms selected from nitrogen, oxygen and sulphur), or a group selected from : 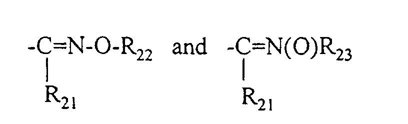 wherein R₂₁ represents a hydrogen atom, a linear or branched (C₁-C₆)-alkyl group or a (C₃-C₇)-cycloalkyl group,
R₂₂ represents a hydrogen atom or a linear or branched (C₁-C₆)-alkyl group, a (C₃-C₇)-cycloalkyl group or a linear or branched (C₁-C₆)-acyl group, R₂₃ represents a linear or branched (C₁-C₆)-alkyl group or a (C₃-C₇)-cycloalkyl group,
R₃ represents a hydrogen atom, a linear or branched (C₁-C₆)-alkyl group, a (C₃-C₇)-cycloalkyl group or a linear or branched (C₁-C₆)-acyl group, or 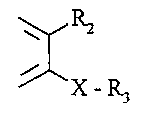 together form the ring 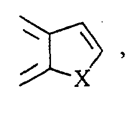
X represents an oxygen atom, a sulphur atom or an NR" group (in which R" represents a hydrogen atom, a linear or branched (C₁-C₆)-alkyl group, a (C₃-C₇)-cycloalkyl group or a linear or branched (C₁-C₆)-acyl group),
the ring B is a phenyl or pyridyl ring,
Ra and Rb, which may be identical or different, represent a hydrogen atom, a halogen atom, a linear or branched (C₁-C₆)-alkyl group, a hydroxy group, a linear or branched (C₁-C₆)-alkoxy group, a carboxy group, a linear or branched (C₁-C₆)-polyhaloalkyl group, a cyano group, a nitro group, a linear or branched (C₁-C₆)-alkoxycarbonyl group, a (C₃-C₇)-cycloalkyloxycarbonyl group, an amino group (optionally substituted by one or two linear or branched (C₁-C₆)-alkyl, linear or branched (C₁-C₆)-acyl, or (C₃-C₇)-cycloalkyl groups), a carbamoyl group (optionally substituted by one or two linear or branched (C₁-C₆)-alkyl, (C₃-C₇)-cycloalkyl or linear or branched (C₁-C₆)-alkoxy groups), a sulpho group, a linear or branched (C₁-C₆)-alkylsulphonyl group, a (C₃-C₇)-cycloalkylsulphonyl group or an aminosulphonyl group (optionally substituted by one or two linear or branched (C₁-C₆)-alkyl or (C₃-C₇)-cycloalkyl groups),
wherein
- when A represents a bond or an oxygen atom, B represents a phenyl ring, R₁ represents a substituted or unsubstituted (C₃-C₇)-cycloalkyl group, a substituted or unsubstituted phenyl group or a substituted or unsubstituted naphthyl group, X represents an oxygen atom or an NR" group (in which R" represents a hydrogen atom or a linear or branched (C₁-C₆)-alkyl group) and R₃ represents a hydrogen atom or a linear or branched (C₁-C₆)-alkyl group, then R₂ is other than a carboxy group,
- substituted alkyl, substituted alkenyl, substituted alkynyl or substituted cycloalkyl group is to be understood as meaning substituted by one or more halogen atoms or linear or branched (C₁-C₆)-alkoxy groups, linear or branched (C₁-C₆)-alkylthio groups, (C₃-C₇)-cycloalkyl groups, unsubstituted or substituted phenyl groups, unsubstituted or substituted naphthyl groups or unsubstituted or substituted heterocyclic groups (such as, for example, a pyridyl group). A preferred saturated or unsaturated substituted alkyl group is the substituted or unsubstituted benzyl group,
- substituted phenyl, substituted naphthyl or substituted heterocyclic or substituted benzyl group is to be understood as meaning substituted by one or more halogen atoms or linear or branched (C₁-C₆)-alkyl, linear or branched (C₁-C₆)-alkoxy or trihalomethyl groups,
their enantiomers, diastereoisomers, and also addition salts thereof with a pharmaceutically acceptable acid or base.

2. Compounds of formula (I) according to claim 1, wherein the ring B is a phenyl ring.

3. Compounds of formula (I) according to claim 1, wherein the ring B is a pyridyl ring.

4. Compounds of formula (I) according to claim 1, wherein X represents an oxygen atom.

5. Compounds of formula (I) according to claim 1, wherein R₁ represents an alkyl group substituted by a substituted or unsubstituted phenyl group.

6. Compounds of formula (I) according to claim 1, wherein R₁ represents an alkyl group substituted by a saturated or unsaturated, substituted or unsubstituted heterocyclic group containing from 1 to 3 hetero atoms selected from oxygen, nitrogen and sulphur.

7. Compounds of formula (I) according to claim 6, wherein R₁ represents an alkyl group substituted by a substituted or unsubstituted pyridyl group.

8. Compounds of formula (I) according to claim 6, wherein R₁ represents a saturated or unsaturated, substituted or unsubstituted heterocyclic group containing from 1 to 3 hetero atoms selected from oxygen, nitrogen and sulphur.

9. Compounds of formula (I) according to claim 1, wherein R₂ represents a halogen atom, a linear or branched, substituted or unsubstituted (C₁-C₆)-alkyl group, a linear or branched, substituted or unsubstituted (C₁-C₆)-alkenyl group, a linear or branched, substituted or unsubstituted (C₁-C₆)-alkynyl group, a substituted or unsubstituted (C₃-C₇)-cycloalkyl group, a formyl group, a carboxy group, a linear or branched (C₁-C₆)-alkylcarbonyl group, a (C₃-C₇)-cycloalkylcarbonyl group, a linear or branched (C₁-C₆)-alkoxycarbonyl group, a (C₃-C₇)-cycloalkyloxycarbonyl group, a carbamoyl group (optionally substituted by one or two groups linear or branched (C₁-C₆)-alkyl, (C₃-C₇)-cycloalkyl, hydroxy, linear or branched (C₁-C₆)-alkoxy, (C₃-C₇)-cycloalkyloxy, or forming together with the nitrogen atom carrying them a ring having from 5 to 7 ring members and containing from 1 to 3 hetero atoms selected from nitrogen, oxygen and sulphur), an amino group (optionally substituted by one or two groups linear or branched (C₁-C₆)-alkyl, linear or branched (C₁-C₆)-acyl, (C₃-C₇)-cycloalkyl, or forming together with the nitrogen atom carrying them a ring having from 5 to 7 ring members and containing from 1 to 3 hetero atoms selected from nitrogen, oxygen and sulphur), a formamidino group, a cyano group, an amidino group (optionally substituted by one or two groups linear or branched (C₁-C₆)-alkyl, (C₃-C₇)-cycloalkyl, or forming together with the nitrogen carrying them a ring having from 5 to 7 ring members and containing from 1 to 3 hetero atoms selected from nitrogen, oxygen and sulphur), a hydroxyaminomethyl group (optionally substituted, independently on the nitrogen or oxygen atom, by a linear or branched (C₁-C₆)-alkyl group, a (C₃-C₇)-cycloalkyl group or a linear or branched (C₁-C₆)-acyl group), an amide oxime group (optionally substituted, independently on the nitrogen or oxygen atom, by a linear or branched (C₁-C₆)-alkyl group, a (C₃-C₇)-cycloalkyl group, or forming with the nitrogen atom carrying them a ring having from 5 to 7 ring members and containing from 1 to 3 hetero atoms selected from nitrogen, oxygen and sulphur), a hydrazono group (optionally substituted by one or two groups linear or branched (C₁-C₆)-alkyl, (C₃-C₇)-cycloalkyl, or forming with the nitrogen atom carrying them a ring having from 5 to 7 ring members and containing from 1 to 3 hetero atoms selected from nitrogen, oxygen and sulphur), or a group selected from 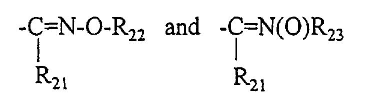 wherein R₂₁ represents a hydrogen atom, a linear or branched (C₁-C₆)-alkyl group or a (C₃-C₇)-cycloalkyl group,
R₂₂ represents a hydrogen atom or a linear or branched (C₁-C₆)-alkyl group, a (C₃-C₇)-cycloalkyl group or a linear or branched (C₁-C₆)-acyl group,
R₂₃ represents a linear or branched (C₁-C₆)-alkyl group or a (C₃-C₇)-cycloalkyl group.

10. Compounds of formula (I) according to claim 1, wherein 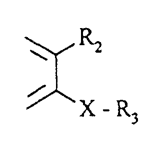 together form the ring

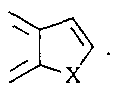

11. Compounds of formula (I) according to claim 10, wherein X represents an oxygen atom.

12. Compound of formula (I) according to claim 1, which is 2-hydroxy-3-(3-nitrophenyl)-5-pyridylbenzaldehyde oxime,
its isomers and also its addition salts with a pharmaceutically acceptable acid or base.

13. Compound of formula (I) according to claim 1, which is 5-benzyl-2-hydroxy-3-(3-nitrophenyl)benzaldehyde oxime,
its isomers and also its addition salts with a pharmaceutically acceptable base.

14. Compound of formula (I) according to claim 1, which is 7-(3-nitrophenyl)-5-(4-pyridylmethyl)benzo[b]furan,
its isomers and also its addition salts with a pharmaceutically acceptable acid.

15. Process for the preparation of compounds of formula (I) according to claim 1, characterised in that there is used as starting material a compound of formula (II): 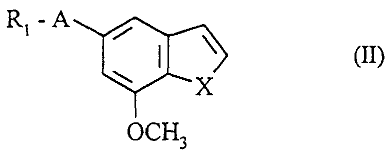 wherein R₁, A and X are as defined for formula (I),
which is subjected to the action of boron tribromide in dichloromethane medium to yield a compound of formula (III): 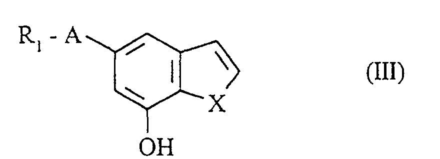 wherein R₁, A and X are as defined for formula (I), which is subjected to the action of trifluoromethanesulphonic anhydride in pyridine to yield a compound of formula (IV): 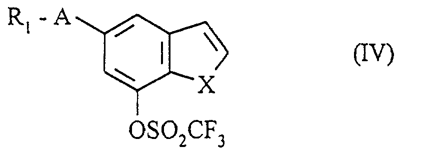 wherein R₁, A and X are as defined for formula (I), which is subjected to the action of a boric acid of formula (V): 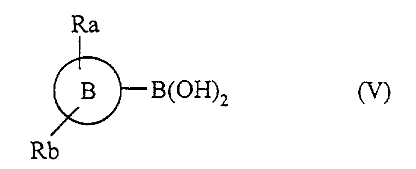 wherein B, Ra and Rb are as defined for formula (I),
to yield a compound of formula (I/a), a particular case of the compounds of formula (I), 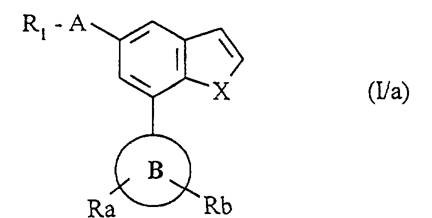 wherein R₁, A, Ra, Rb, B et X are as defined for formula (I),
which compound of formula (I/a) is, if desired, subjected to oxidative cleavage to yield a compound of formula (I/b), a particular case of the compounds of formula (I): 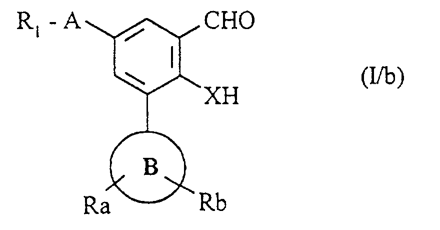 wherein R₁, A, Ra, Rb, B and X are as defined for formula (I),
which compound of formula (I/b) may be subjected :
**a** to the action of a compound of formula (VI) :
R₂₂O - NH₂ (VI)
wherein R₂₂ represents a hydrogen atom, a linear or branched (C₁-C₆)-alkyl group, a (C₃-C₇)-cycloalkyl group or a linear or branched (C₁-C₆)-acyl group, in basic medium,
to yield a compound of formula (I/c), a particular case of the compounds of formula (I): 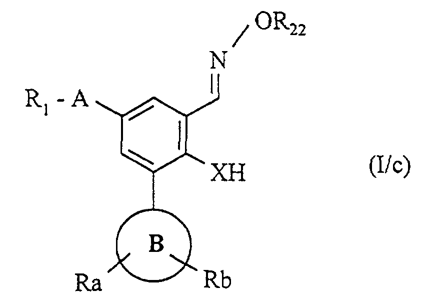 wherein R₁, A, Ra, Rb, B, X and R₂₂ are as defined hereinbefore,
which compound of formula (I/c), when R₂₂ is other than an acyl group, is optionally converted in acid medium to a compound of formula (I/d), a particular case of the compounds of formula (I): 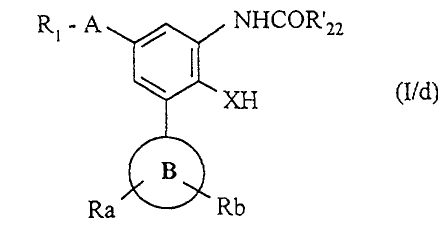 wherein R₁, A, Ra, Rb, B and X are as defined hereinbefore and R'₂₂, which is other than an acyl group, has the same meanings as R₂₂,
**b** or to the action, in alcoholic medium, of a hydroxylamine of formula R₂₃NH-OH wherein R₂₃ represents a linear or branched (C₁-C₆)-alkyl group or a (C₃-C₇)-cycloalkyl group to yield a compound of formula (I/e), a particular case of the compounds of formula (I) : 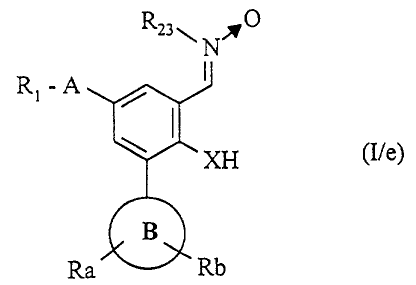 wherein R₁, A, B, Ra, Rb, X and R₂₃ are as defined hereinbefore,
**c** or to the action of a phosphorus ylid of formula R'cCH₂P⁺(C₆H₅)₃Br⁻ (wherein R'c represents a hydrogen atom, a linear or branched (C₁-C₆)-alkyl group, a (C₃-C₇)-cycloalkyl group, a linear or branched (C₁-C₆)-alkoxy group, a linear or branched (C₁-C₆)-alkylthio group, a substituted or unsubstituted phenyl group, a substituted or unsubstituted naphthyl group or a saturated or unsaturated, substituted or unsubstituted heterocyclic group) in the presence of n-butyllithium,
to yield a compound of formula (I/f), a particular case of the compounds of formula (I): 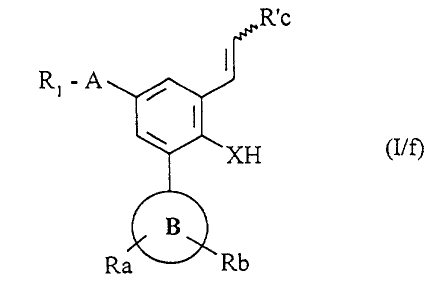 wherein R₁, A, Ra, Rb, B, X and R'c are as defined hereinbefore,
which, if desired, is subjected to reduction to yield a compound of formula (I/g), a particular case of the compounds of formula (I): 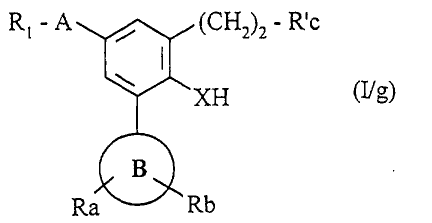 wherein R₁, A, Ra, Rb, B, X et R'c are as defined hereinbefore,
**d** or to a reaction in oxidising medium to yield a compound of formula (I/h): 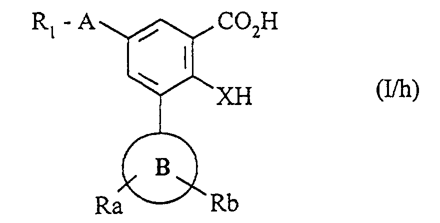 wherein R₁, A, Ra, R, B and X are as defined for formula (I),
the acid function of which compound of formula (I/h) is converted into the corresponding acid chloride, which may then be converted into the corresponding ester, amide or amine according to conventional reactions in organic chemistry,
**e** or to the action of hydroxylamine hydrochloride in an appropriate medium to yield a compound of formula (I/i), a particular case of the compounds of formula (I): 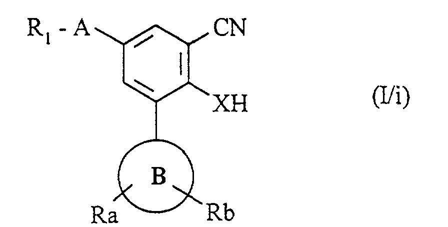 wherein R₁, A, Ra, Rb, B and X are as defined hereinbefore,
- which is reacted in alcoholic medium with hydrochloric acid, and then with an amine Rd-NH-Re (wherein Rd and Re, which may be identical or different, represent a hydrogen atom, a linear or branched (C₁-C₆)-alkyl group or a cycloalkyl group, or form together with the nitrogen atom carrying them a ring having from 5 to 7 ring members and containing from 1 to 3 hetero atoms selected from nitrogen, oxygen and sulphur),
to yield a compound of formula (I/j), a particular case of the compounds of formula (I): 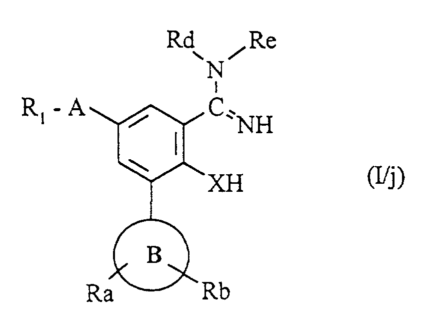 wherein R₁, A, Ra, Rb, X, B, Rd and Re are as defined hereinbefore,
- or is reacted in basic medium with a compound of formula (VI):
RcO-NH₂ (VI)
wherein Rc is as defined for formula (I),
to yield a compound of formula (I/k), a particular case of the compounds of formula (I): 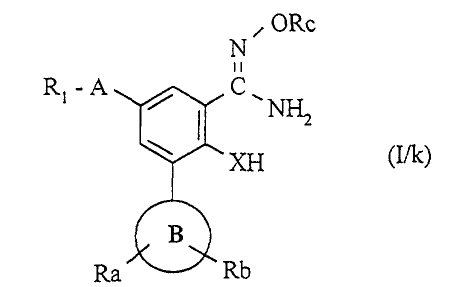 wherein A, B, R₁, Ra, Rb, Rc and X are as defined hereinbefore,
**f** or, finally, with a hydrazine 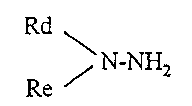 (Rd and Re being as defined hereinbefore) to yield a compound of formula (I/I), a particular case of the compounds of formula (I) : 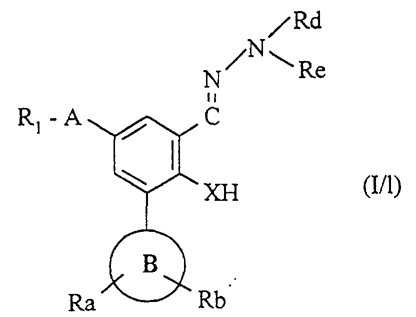 wherein A, B, R₁, Ra, Rb, Rd, Re and X are as defined hereinbefore,
which compound of formula (I/a), (I/b), (I/c), (I/d), (I/e), (I/f), (I/g), (I/h), (I/i), (I/l) or derivatives thereof:
- are, if desired, subjected to the action of a dialkyl sulphate or a linear or branched (C₁-C₆)-alkyl halide or a linear or branched (C₁-C₆)-acyl halide or a (C₃-C₇)-cycloalkyl halide,
to yield a compound of formula (I/m), a particular case of the compounds of formula (I): 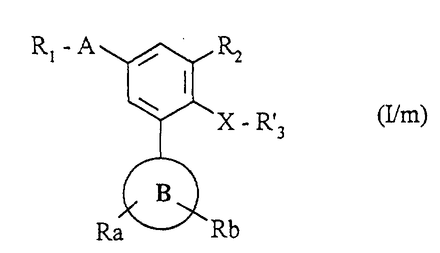 wherein R₁, A, B, Ra, Rb, R₂ and X are as defined hereinbefore and R'₃ represents a linear or branched (C₁-C₆)-alkyl group, a linear or branched (C₁-C₆)-acyl group or a (C₃-C₇)-cycloalkyl group,
which compound of formula (I/a) to (I/m) or derivatives thereof:
- may, if necessary, be purified according to a conventional purification technique,
- is, if desired, separated into the isomers according to a conventional separation technique,
- is, if desired, converted into its addition salts with a pharmaceutically acceptable acid or base.

16. Process for the preparation of compounds of formula (I) according to claim 1, characterised in that there is used as starting material a compound of formula (XI) : 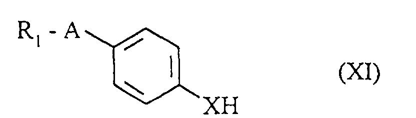 wherein X, A and R₁ are as defined for formula (I), which is subjected to a bromination reaction to yield a compound of formula (XII) 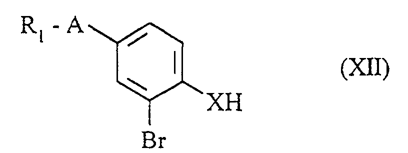 wherein X, A and R₁ are as defined hereinbefore,
which is subjected to the action of a boric acid of formula (V): 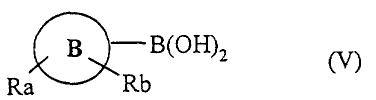 wherein Ra, Rb and B are as defined for formula (I),
to yield a compound of formula (XIII) : 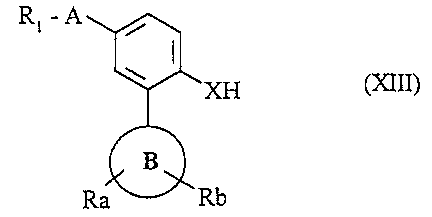 wherein A, R₁, Ra, Rb, B and X are as defined hereinbefore,
which compound (XIII) is subjected to a halogenation reaction to yield a compound of formula (I/n), a particular case of the compounds of formula (I) : 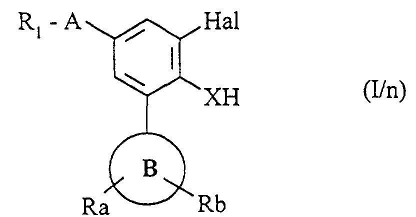 wherein A, R₁, Ra, Rb, B and X are as defined hereinbefore and Hal represents a halogen atom,
which compound (I/n) may be subjected :
- either to the action of zinc cyanide in the presence of a palladium catalyst to yield a compound of formula (I/i) as defined hereinbefore,
- or to the action of a tin compound in the presence of a palladium catalyst to yield a compound of formula (I/p), a particular case of the compounds of formula (I): 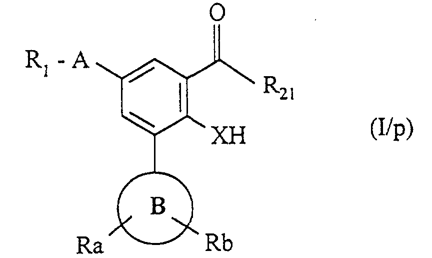 wherein A, R₁, Ra, Rb, B and X are as defined for formula (I) and R₂₁, which is other than a hydrogen atom, is as defined for formula (I),
which compound (I/p) is reacted :
- either with a compound of formula (VI) :
R₂₂O-NH₂ (VI)
wherein R₂₂ represents a hydrogen atom or a linear or branched (C₁-C₆)-alkyl, a (C₃-C₇)-cycloalkyl or a linear or branched (C₁-C₆)-acyl group,
to yield a compound of formula (I/q), a particular case of the compounds of formula (I) : 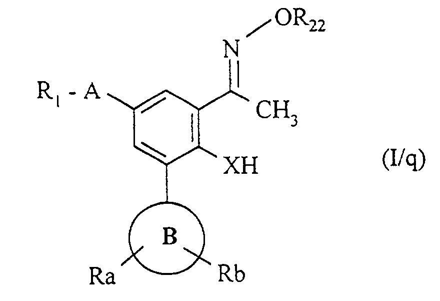 wherein A, B, R₁, Ra, Rb, R₂₁, R₂₂ and X are as defined hereinbefore;
- or with a hydrazine 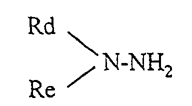 wherein Rd, Re, which may be identical or different, represent a hydrogen atom or a group linear or branched (C₁-C₆)-alkyl or a cycloalkyl group or form together with the nitrogen atom carrying them a ring having from 4 to 7 ring members and containing from 1 to 3 hetero atoms selected from nitrogen, oxygen and sulpur, to yield a compound of formula (I/s), a particular case of the compounds of formula (I): 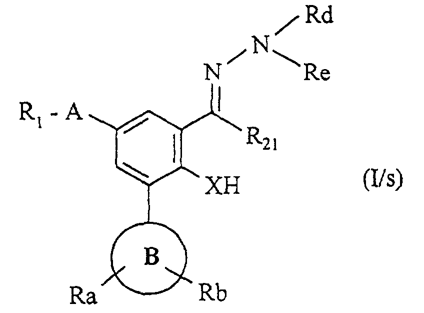 wherein A, B, R₁, Ra, Rb, Rd, Re, R₂₁ and X are as defined hereinbefore,
which compounds (I/n), (I/p), (I/q), (I/s) or derivatives thereof are optionally subjected to the action of a dialkyl sulphate or a linear or branched (C₁-C₆)-alkyl halide, a linear or branched (C₁-C₆)-acyl halide or a (C₃-C₇)-cycloalkyl halide to yield a compound of formula (I/m) as defined hereinbefore,
which compounds (I/n) to (I/s) or derivatives thereof:
- may, if necessary, be purified according to a conventional purification technique,
- are, if desired, separated into isomers according to a conventional separation technique,
- are, if desired, converted into addition salts with a pharmaceutically acceptable acid or base.

17. Pharmaceutical compositions comprising as active ingredient at least one compound according to any one of claims 1 to 14, alone or in combination with one or more pharmaceutically acceptable, inert, non-toxic excipients or carriers.

18. Pharmaceutical compositions according to claim 17 comprising at least one active ingredient according to any one of claims 1 to 14 for use as inhibitors of group IV phosphodiesterases.
